# EUROPEAN PATENT APPLICATION

(11) **EP 4 368 717 A1**
(43) Date of publication of application: **15.05.2024**
(21) Application number: 22838056.4
(22) Date of filing: 08.07.2022
(51) Int. Cl.: C12N 15/113, C07H 19/048

(54) **RNAI AGENT TARGETING MARC1 GENE, AND USE THEREOF**

(30) Priority: 08.07.2021 KR 20210089864
(71) Applicant: Olix Pharmaceuticals, Inc., Suwon-si, Gyeonggi-do 16226 (KR)
(72) Inventor: HONG, Sun Woo, Yongin-si, Gyeonggi-do 16822 (KR); PARK, June Hyun, Suwon-si, Gyeonggi-do 16281 (KR); CHOE, Jeong Yong, Suwon-si, Gyeonggi-do 16295 (KR)
(74) Representative: Weickmann & Weickmann PartmbB
(86) International application number: PCT/KR2022/009984
(87) International publication number: WO 2023/282704

(57) **Abstract**

The present disclosure relates to an RNAi agent targeting a mitochondrial amidoxime reducing component 1 (MARC1) gene and a use thereof, specifically, the present disclosure relates an RNAi agent including an antisense strand having sequence complementarity to a MARC1 mRNA sequence and a sense strand having sequence complementarity to the antisense strand, and a pharmaceutical composition including the RNAi agent for preventing or treating liver disease, such as non-alcoholic fatty liver disease.

## Description

### TECHNICAL FIELD

The present disclosure relates to an RNAi agent targeting a mitochondrial amidoxime reducing component 1 (MARC1) gene and a use thereof, specifically, the present disclosure relates an RNAi agent including an antisense strand having sequence complementarity to a MARC1 mRNA sequence and a sense strand having sequence complementarity to the antisense strand, and a pharmaceutical composition including the RNAi agent for preventing or treating liver disease, such as non-alcoholic fatty liver disease.

### BACKGROUND ART

Recently, prevalence of non-alcoholic fatty liver disease is increasing along with increasing prevalence of metabolic syndrome due to an increase in obese population. Fatty liver is a disease in which triglycerides are accumulated in hepatocytes and is reported to be a complication of obesity or caused by various other causes, such as alcohol, diabetes, malnutrition, and drug abuse. Fatty liver disease is divided into alcoholic fatty liver disease caused by excessive alcohol intake and non-alcoholic fatty liver disease (NAFLD), in which triglycerides are accumulated in the liver regardless of alcohol intake, and nonalcoholic fatty liver disease includes simple steatosis and nonalcoholic steatohepatitis (NASH).

Simple steatosis has a relatively good prognosis, but when fat accumulation becomes severe, the disease progresses to inflammation, that is, steatohepatitis. In the liver with extensive steatohepatitis, necrosis of hepatocytes appears, and stellate cells are activated to cause fibrosis, in particular, severe perisinusoidal fibrosis at the end of the hepatic vein occurs. Therefore, if steatohepatitis is left unattended, it may develop into liver fibrosis or cirrhosis, resulting in poor prognosis. 15 % to 50 % of patients with nonalcoholic steatohepatitis develop hepatic fibrosis or cirrhosis, and 30 % of patients with fibrosis develop cirrhosis after 10 years.

Nonalcoholic fatty liver disease is closely related to various metabolic syndromes such as obesity, heart disease, and diabetes, and in this regard, drugs with inhibitory effects on metabolic syndrome, such as insulin resistance improver, antioxidants, hyperlipidemia drugs, hepatic protectant, and angiotensin II receptor antagonists, have been clinically tried. However, to date, drugs with clinical evidence for treatment of non-alcoholic fatty liver disease have not been developed, and all drugs currently in use are off-label drugs that may be selected as suboptimal solutions, but these existing off-label drugs lack clinical evidence and have limitations in their use due to safety issues, etc. For example, Pioglitazone, an insulin resistance improver, had been considered promising as a medicine for nonalcoholic steatohepatitis, however, actual improvement effect on liver fibrosis did not appear in clinical practice, and due to side effects such as fracture risk, weight gain, and worsening and onset of heart failure, criteria for a therapeutic agent were not clearly satisfied. In this situation where there is no approved medicine for nonalcoholic fatty liver disease, recently, the US Food and Drug Administration (FDA) has suggested reduction of inflammation, reduction of liver fibrosis, and alleviation of symptoms of nonalcoholic steatohepatitis as clinical indicators of nonalcoholic steatohepatitis.

On the other hand, treatment of diseases using the phenomenon of RNA interference is attracting attention as a safer treatment for diseases, since the treatment uses small interfering RNA (siRNA) that targets mRNA and regulates gene expression at a translational level.

Accordingly, the present inventors developed an RNA agent using RNA interference technology as a result of earnest research efforts to develop a new safe drug that exhibits effects of improvement in regard to clinical indicators of nonalcoholic steatohepatitis.

### DESCRIPTION OF EMBODIMENTS

### TECHNICAL PROBLEM

An object of the present disclosure is to provide an RNAi agent that specifically inhibits expression of MARC1.

Another object of the present disclosure is to provide a pharmaceutical composition including the RNAi agent for preventing or treating liver disease, or a method of preventing or treating liver disease.

### SOLUTION TO PROBLEM

In order to achieve the above objects, an aspect provides an RNAi agent including: an antisense strand having sequence complementarity to the mRNA sequence of mitochondrial amidoxime reducing component 1 (MARC1), and is 19 nt to 21 nt in length (nt); and a sense strand having sequence complementarity to the antisense strand, and is 15 nt to 17 nt in length, wherein the 5' end of the antisense strand and the 3' end of the sense strand form a blunt end.

Another aspect provides a pharmaceutical composition including the RNAi agent as an active ingredient for preventing or treating liver disease.

Still another aspect provides a method of preventing or treating liver disease, including administering the RNAi agent to a subject.

Still another aspect provides a use of the RNAi agent for manufacturing a pharmaceutical product for preventing or treating liver disease.

### ADVANTAGEOUS EFFECTS OF DISCLOSURE

An RNAi agent according to an aspect may inhibit expression of MARC1 by binding to and degrading the mRNA encoding MARC1 while alleviating side effects such as non-specific immune responses and off-target effects.

In addition, the RNAi agent according to an aspect exhibits effects such as alleviation of fat accumulation in liver tissue, reduction of inflammation, reduction of liver fibrosis, and alleviation of symptoms of nonalcoholic steatohepatitis, by targeting the hepatocyte surface receptor, and therefore, may be useful as a targeted therapy for liver diseases including non-alcoholic fatty liver disease, liver fibrosis, and liver cirrhosis.

### BRIEF DESCRIPTION OF DRAWINGS

FIG. 1 shows results of confirming expression levels of MARC1 mRNA, after 50 types of MARC1 asiRNA according to an aspect are treated to Huh7 cells each at a concentration of 10 nM.
FIG. 2 shows results of confirming expression levels of MARC1 mRNA, after treating primary hepatocytes derived from a C57BL/6 mouse with 23 types of MARC1 GalNAc-asiRNAs according to an aspect each at a concentration of 200 nM.
FIG. 3 shows results of confirming expression levels of MARC1 and MARC2 mRNA, after administration of OLX-003-1 or OLX-031-1 according to an aspect to animal models provided with a high-fat diet (#3-1, #31-1); FIG. 3A shows results of confirming expression levels of MARC1 mRNA, and FIG. 3B shows results of confirming expression levels of MARC2 mRNA.
FIG. 4 shows results of visually confirming appearances of the liver, after administration of OLX-003-1 or OLX-031-1 according to an aspect to animal models provided with a high-fat diet (#3-1, #31-1).
FIG. 5 shows results of confirming levels of fat vacuoles in the liver parenchyma through H&E staining, after administration of OLX-003-1 or OLX-031-1 according to an aspect to animal models provided with a high-fat diet (#3-1, #31-1).
FIG. 6 shows results of confirming levels of fat vacuoles in the liver parenchyma through Oil Red O staining, after administration of OLX-003-1 or OLX-031-1 according to an aspect to animal models provided with a high-fat diet (#3-1, #31-1).
FIG. 7 confirms expression levels of MARC1 and MARC2 mRNA, after administration of OLX-031-1 according to an aspect to animal models provided with a high-fat diet (#031-1); FIG. 7A shows results of confirming expression levels of MARC1 mRNA, and FIG. 7B shows results of confirming expression levels of MARC2 mRNA.
FIG. 8 shows results of visually confirming appearances of the liver, after administration of OLX-031-1 according to an aspect to animal models provided with a high-fat diet (#031-1).
FIG. 9 shows results of confirming levels of fat vacuoles in the liver parenchyma through H&E staining, after administration of OLX-031-1 according to an aspect to animal models provided with a high-fat diet (#031-1).
FIG. 10 shows results of confirming levels of fat vacuoles and collagen deposition in the liver parenchyma through Picro Sirius Red staining, after administration of OLX-031-1 according to an aspect to animal models provided with a high-fat diet (#31-1).
FIG. 11 shows results of evaluation of expression levels of liver fibrosis-related factors, after administration of OLX-031-1 according to an aspect to animal models provided with a high-fat diet (#031-1); FIG. 11A shows results of confirming expression levels of α-SMA mRNA, and FIG. 11B shows results of confirming expression levels of Col1α1 mRNA.
FIG. 12 shows results of confirming levels of triglycerides in liver tissue, after administration of OLX-031-1 according to an aspect to animal models provided with a high-fat diet (#031-1).
FIG. 13 confirms change in liver damage index factors, after administration of OLX-031-1 according to an aspect to animal models provided with a high-fat diet (#031-1); FIG. 13A shows results of confirming aspartate aminotransferase (AST) levels in the serum, FIG. 13B shows results of confirming alanine aminotransferase (ALT) levels in the serum, and FIG. 13C shows results of calculating ratio of AST/ALT in the serum.
FIG. 14 confirms changes in lipid indicators in the serum, after administration of OLX-031-1 according to an aspect to animal models provided with a high-fat diet (#31-1); FIG. 14A shows results of confirming levels of cholesterol, FIG. 14B shows results of confirming levels of triglycerides, FIG. 14C shows results of confirming levels of low-density lipoprotein, and FIG. 14D shows results of confirming levels of high-density lipoprotein.
FIG. 15 shows results of confirming levels of fatty vacuoles and inflammatory foci in the liver parenchyma by H&E staining, after administration of OLX-031-2 according to an aspect to animal models provided with a high-fat diet (CDHFD 031-2, CDHFD-NCD 031-2).
FIG. 16 shows results of confirming levels of fat vacuoles and collagen deposition in the liver parenchyma through Picro Sirius Red staining, after administration of OLX-031-2 according to an aspect to animal models provided with a high-fat diet (CDHFD 031 - 2, CDHFD-NCD 031-2).
FIG. 17 shows results of comparing contents of collagen components in the liver parenchyma, by comparing PSR staining area (%), after administration of OLX-031-2 according to an aspect to animal models provided with a high-fat diet (CDHFD 031-2, CDHFD-NCD 031-2).
FIG. 18 shows results of evaluation of expression levels of liver fibrosis-related factors, after administration of OLX-031-2 according to an aspect to animal models provided with a high-fat diet (CDHFD 031-2, CDHFD-NCD 031-2); FIG. 18A shows results of confirming expression levels of Col1α1 mRNA, FIG. 18B shows results of confirming expression levels of α-SMA mRNA, and FIG. 18C shows results of confirming expression levels of TIMP1 mRNA.
FIG. 19 shows results of confirming expression levels of MARC1 mRNA, after administration of OLX-031-2 according to an aspect to animal models provided with a high-fat diet (CDHFD 031-2 and CDHFD-NCD 031-2).
FIGS. 20A, 20B, and 20C show results of confirming expression levels of MARC1 mRNA, after Huh7 cells are treated with 134 types of MARC1 asiRNAs according to an aspect each at a concentration of 1 nM.
FIG. 21 shows results of confirming expression levels of MARC1 mRNA, after 41 types of MARC1 asiRNAs according to an aspect are treated to Huh7 cells, each at a concentration of 0.1 nM.
FIG. 22 shows results of confirming expression levels of MARC1 protein after 30 types of MARC1 asiRNAs according to an aspect are treated to Huh7 cells, each at a concentration of 1 nM.
FIG. 23 shows results of confirming expression levels of MARC1 mRNA, after 41 types of MARC1 GalNAc-asiRNAs according to an aspect are treated to primary mouse hepatocytes, each at a concentration of 100 nM.
FIG. 24 shows results of confirming expression levels of MARC1 mRNA, after 41 types of MARC1 GalNAc-asiRNAs according to an aspect are treated to human-derived primary hepatocytes, each at a concentration of 500 nM.
FIG. 25 shows results of confirming expression levels of MARC1 mRNA, after 41 types of MARC1 GalNAc-asiRNAs according to an aspect are treated to human-derived primary hepatocytes, each at a concentration of 20 nM or 100 nM.
FIG. 26 shows results of confirming expression levels of MARC1 mRNA, after 17 types of MARC1 GalNAc-asiRNAs according to an aspect are treated to human-derived primary hepatocytes, each at a concentration of 10 nM or 100 nM.
FIG. 27 shows results of confirming expression levels of MARC1 mRNA, after administering OLX-031-2 or OLX-075-2 according to an aspect to monkeys, each at a concentration of 2.5 mpk, 5 mpk, or 10 mpk.
FIG. 28 shows results of confirming expression levels of MARC1 mRNA through levels of SEAP reporter fluorescence, after administering 9 types of MARC1 GalNAc-asiRNAs to animal models transfected with pSELECT-mSEAP-hMARC1.
FIG. 29 shows results of confirming expression levels of MARC1 mRNA through levels of luciferase reporter fluorescence, after administration of 10 types of MARC1 GalNAc-asiRNAs to animal models transfected with pSELECT-mSEAP-hMARC1.

### MODE OF DISCLOSURE

Each description and embodiment disclosed in the present application may also be applied to other descriptions and embodiments. That is, all combinations of the various elements disclosed in the application fall within the scope of the application. In addition, it should not be construed that the scope of the present application is limited by the detailed description described below.

An aspect provides an RNAi agent including: an antisense strand having sequence complementarity to the mRNA sequence of mitochondrial amidoxime reducing component 1 (MARC1), and is 19 nt to 21 nt in length (nt); and a sense strand having sequence complementarity to the antisense strand, and is 15 nt to 17 nt in length, wherein the 5' end of the antisense strand and the 3' end of the sense strand form a blunt end.

### RNAi agent

The term "RNA interference (RNAi)" refers to a mechanism for suppressing expression of a target gene by inducing degradation of mRNA of the target gene by introduction of double-stranded RNA (dsRNA) composed of a strand having a sequence homology to the target gene mRNA and a strand having a sequence complementary to the aforementioned strand.

The term "RNAi agents" or "nucleic acid molecules inducing RNAi", used herein, refers to any agent or nucleic acid molecule that is capable of inhibiting or downregulating gene expression or viral replication by mediating the RNA interference in a sequence-specific manner. The term may refer to both an individual nucleic acid molecule, a plurality of the nucleic acid molecules, or a pool of the nucleic acid molecules. In an embodiment, the RNAi agent may be siRNA.

The term "small interfering RNA (siRNA; short interfering RNA)" refers to a short double-stranded RNA (dsRNA) that sequence-specifically mediates efficient gene silencing.

The term "gene", used herein, should be considered in its broadest sense, and may encode a structural protein or a regulatory protein. In this regard, the regulatory protein includes a transcription factor, a heat shock protein, or a protein involved in DNA/RNA replication, transcription, and/or translation. In the present disclosure, the target gene to be suppressed is inherent in the viral genome, and may be integrated into an animal genome or exist as an extrachromosomal component.

The term "antisense strand", used herein, refers to a polynucleotide that is substantially or 100 % complementary to a target nucleic acid of interest, and may be complementary in whole or in part with, for example, messenger RNA (mRNA), non-mRNA RNA sequences (e.g., microRNA, piwiRNA, tRNA, rRNA and hnRNA), or coding or non-coding DNA sequences.

The term "sense strand", used herein, refers to a polynucleotide having the same nucleic acid sequence as a target nucleic acid, or a polynucleotide in whole or in part the same with, for example, messenger RNA (mRNA), non-mRNA RNA sequences (e.g., microRNA, piwiRNA, tRNA, rRNA and hnRNA), or coding or non-coding DNA sequences.

The term "complementarity" or "complementary", used herein, refers to a generally accepted meaning in the art. The term generally may refer to formation or presence of hydrogen bond(s) between one nucleic acid sequence and another nucleic acid sequence, either by traditional Watson-Crick bonding or other non-traditional types of bonding as described herein. Perfect complementarity may mean that all contiguous residues of a nucleic acid sequence hydrogen bond with the same number of contiguous residues in a second nucleic acid sequence. Partial complementarity within a nucleic acid molecule may include various mismatches or non-base-paired nucleotides (e.g., 1, 2, 3, 4, 5, 6, 7, 8, 9, or 10 or more mismatches, e.g., 1 to 3 mismatches, non-nucleotide linkers, or non-base pair nucleotides). The partial complementarity may result in bulges, loops, overhangs, or blunt ends between a sense strand and an antisense strand of a nucleic acid molecule, or between an antisense strand of a nucleic acid molecule and a corresponding target nucleic acid molecule.

The term "blunt end", used herein, refers to a generally accepted meaning in the art. In relation to the RNAi agents or nucleic acid molecules herein, the term may refer to an end of a double-stranded siRNA molecule that lacks overhanging nucleotides. The siRNA molecules described herein may be such that the 5'-end of an antisense strand and the 3'-end of a sense strand form a blunt end.

### RNAi Agents for silencing expression of MARC1

"Mitochondrial amidoxime reducing component 1 (MARC1)" is a mammalian enzyme containing molybdenum, also referred to as MTARC1 or MOSC1, and it is known that MARC1 enzyme deficiency is associated with low blood cholesterol level, low blood liver enzyme level, reduced liver fat, and reduced risk of developing cirrhosis, making it a potential therapeutic target for liver disease. The MARC1 protein may be interpreted to include the naturally occurring wild-type MARC1 and functional variants thereof, and the sequence of the MARC1 protein or a gene encoding the same may be obtained from a known database such as GenBank of the US National Institutes of Health.

The term "expression", used herein, refers to the generally accepted meaning in the art. The term may generally refer to a process by which a gene ultimately produces a protein. The expression includes, but is not limited to, transcription, splicing, post-transcriptional modification, or translation. As used herein, an expression level may be determined or monitored by detection of an mRNA level or a protein level.

The term "inhibition" or "reduction", as used in reference to MARC1 gene expression in a subject, refers to a statistically significant decrease compared to an untreated or normal control group. The reduction may be, for example, at least 30 %, 35 %, 40 %, 45 %, 50 %, 55 %, 60 %, 60 %, 65 %, 70 %, 70 %, 80 %, 85 %, 90 %, or 95 % or more, but it may be below the detection limit, depending on the detection or measurement method.

siRNA is a small interfering RNA and is involved in RNA interference (RNAi). RNAi is an intracellular gene regulation mechanism first discovered in 1998 in *Caenorthabditis elegans*, and its mechanism of action is known to induce target gene degradation by an antisense strand, which is one of RNA double strands which are introduced into the cell, that complementarily binds to mRNA of a target gene, and RNAi has recently been one of the most popular candidate technologies for developing new drugs.

However, contrary to this possibility, side effects and disadvantages of siRNA have been continuously reported. For the development of RNAi-based therapeutic agents, it is necessary to overcome barriers such as 1) absence of an effective delivery system, 2) off-target effect, 3) induction of immune response, and 4) saturation of RNAi machinery in cells. Although siRNA is an effective method for directly regulating expression of a target gene, it is difficult to develop a therapeutic agent due to these issues. In this regard, asymmetric shorter duplex siRNA (asiRNA) is an asymmetric RNAi-induced structure having a shorter double helix length compared to the 19+2 structure of siRNA in the art. asiRNA technology overcame issues such as off-target effect, saturation of RNAi mechanism, and immune response by TLR3, which are identified in the existing siRNA structure technology, and accordingly, it is possible to develop new RNAi drugs with low side effects.

Based on this, asymmetric siRNA including a sense strand and an antisense strand complementary to the sense strand is presented in this example, and siRNA according to an example does not cause issues such as off-target effect, saturation of RNAi machinery, etc., and thereby, may inhibit expression of an MARC1 gene to a desired degree while stably maintaining high delivery efficiency.

In an example, asymmetric siRNAs (asiRNAs) targeting MARC1 were designed and prepared, and after transfection of the asiRNAs into cells expressing MARC1, nucleic acid molecules for inducing RNAi with excellent knockdown efficiency, i.e., MARC1 asiRNAs, were selected.

In an embodiment, the RNAi agent may be characterized in that the sense strand has a length of 15 nt to 17 nt, and the antisense strand has a length of 19 nt to 21 nt. More preferably, a length of the sense strand may be 16 nt, and a length of the antisense strand complementary thereto may be 19 nt, 20 nt, or 21 nt, but is not limited thereto.

The 5'-end of the antisense strand and the 3'-end of the sense strand may form a blunt end. The 3' end of the antisense strand may include, for example, an overhang of 2 nt to 6 nt.

In an embodiment, the sense strand may be a sequence selected from the sense strand sequences listed in Table 1, Table 10, Table 11, Table 12, Table 13, and Table 14, and the antisense strand may include a sequence selected from the antisense strand sequences listed in Table 1, Table 10, Table 11, Table 12, Table 13, and Table 14.

In an embodiment, the sense strand may be, for example, any one selected from the group consisting of SEQ ID NO: 149, SEQ ID NO: 159, SEQ ID NO: 177, SEQ ID NO: 179, SEQ ID NO: 183, SEQ ID NO: 197, SEQ ID NO: 199, SEQ ID NO: 227, SEQ ID NO: 247, SEQ ID NO: 251, SEQ ID NO: 257, SEQ ID NO: 259, SEQ ID NO: 261, SEQ ID NO: 273, SEQ ID NO: 285, SEQ ID NO: 287, SEQ ID NO: 331, SEQ ID NO: 353, SEQ ID NO: 387, SEQ ID NO: 391, SEQ ID NO: 399, SEQ ID NO: 423 , SEQ ID NO: 429, SEQ ID NO: 431, SEQ ID NO: 435, SEQ ID NO: 439, SEQ ID NO: 443, SEQ ID NO: 445, SEQ ID NO: 447, and SEQ ID NO: 527, or for example, any one selected from the group consisting of SEQ ID NO: 61, SEQ ID NO: 149, SEQ ID NO: 183, SEQ ID NO: 227, SEQ ID NO: 423, SEQ ID NO: 431, SEQ ID NO: 435, SEQ ID NO: 439, SEQ ID NO: 447, SEQ ID NO: 527, and SEQ ID NO: 551.

In an embodiment, the antisense strand may be, for example, any one selected from the group consisting of SEQ ID NO: 150, SEQ ID NO: 160, SEQ ID NO: 178, SEQ ID NO: 180, SEQ ID NO: 184, SEQ ID NO: 198, SEQ ID NO: 200, SEQ ID NO: 228, SEQ ID NO: 248, SEQ ID NO: 252, SEQ ID NO: 258, SEQ ID NO: 260, SEQ ID NO: 262, SEQ ID NO: 274, SEQ ID NO: 286, SEQ ID NO: 288, SEQ ID NO: 332, SEQ ID NO: 354, SEQ ID NO: 388, SEQ ID NO: 392, SEQ ID NO: 400, SEQ ID NO: 424 , SEQ ID NO: 430, SEQ ID NO: 432, SEQ ID NO: 436, SEQ ID NO: 440, SEQ ID NO: 444, SEQ ID NO: 446, SEQ ID NO: 448, and SEQ ID NO: 528, or for example, may be any one selected from the group consisting of SEQ ID NO: 62, SEQ ID NO: 150, SEQ ID NO: 184, SEQ ID NO: 228, SEQ ID NO: 424, SEQ ID NO: 432, SEQ ID NO: 436, SEQ ID NO: 440, SEQ ID NO: 448, SEQ ID NO: 528, and SEQ ID NO: 552.

### RNAi agent with chemical modifications introduced

In the RNAi agent, the sense strand or the antisense strand may include one or more chemical modifications.

General siRNA cannot pass through the cell membrane due to high negative charge and high molecular weight of the phosphate backbone structure, and is rapidly degraded and eliminated from the blood, making it difficult to deliver a sufficient amount to an actual target site for RNAi induction. Currently, in case of *in vitro* delivery, many high-efficiency delivery methods using cationic lipids and cationic polymers have been developed, however, in case of *in vivo*, it is difficult to deliver siRNA with as high efficiency as *in vitro*, and there is an issue of reduced siRNA delivery efficiency due to interaction with various proteins existing in the living body.

Accordingly, provided in the example is an RNAi agent having improved hepatocyte targeted delivery ability by introduction of chemical modifications to the asymmetric siRNA structure, more specifically, provided is an asymmetric siRNA construct (GalNAc asymmetric siRNA, GalNAc-asiRNA) capable of effective intracellular delivery without a separate carrier.

In the present disclosure, the chemical modification in the sense strand or the antisense strand may include one or more selected from the group consisting of: binding with an N-acetylgalactosamine (GalNAc) derivative; modification of a nucleotide bond with phosphorothioate, boranophosphate, or methyl phosphonate; substitution of an -OH group at the 2' carbon position of the sugar structure of a nucleotide with -CH₃ (methyl), - OCH₃ (methoxy), -NH₂, -F, -O-2-methoxyethyl-O-propyl, -O-2-methyl methylthioethyl, -O-3-aminopropyl, or -O-3-dimethylaminopropyl; and binding of a phosphate group, E-vinylphosphonate, or a cell penetrating peptide.

In an embodiment, the N-acetylgalactosamine (GalNAc) derivative may have a structure of Formula 1 below. The N-acetylgalactosamine (GalNAc) derivative recognizes asialoglycoprotein (ASGPR) receptors on the surface of a hepatocyte and helps RNAi agents to flow into the hepatocyte, that is, acts as an ASGPR targeting moiety, and thus, the RNAi agent, in which the N-acetylgalactosamine (GalNAc) derivative is bound to an end have improved delivery to hepatocytes and may provide an effective targeted treatment for liver diseases.

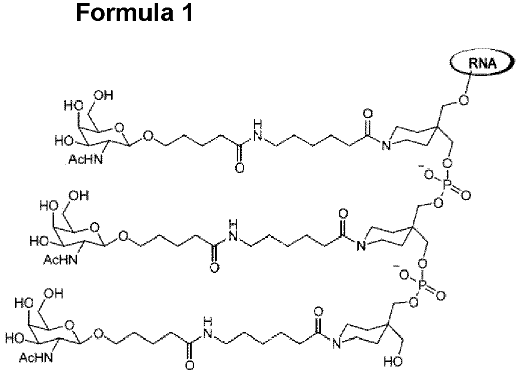

In an embodiment, the sense strand may include one or more chemical modifications selected from: modification of 2 to 4 nucleotide bonds adjacent to the 5' end with phosphorothioate, boranophosphate, or methyl phosphonate; substitution of an -OH group at the 2' carbon position of the sugar structure of at least one nucleotide with -CH₃ (methyl), -OCH₃ (methoxy), -NH₂, -F, -O-2-methoxyethyl-O-propyl, -O-2-methyl methylthioethyl, -O-3-aminopropyl, or -O-3-dimethylaminopropyl; and binding with an N-acetylgalactosamine (GalNAc) derivative, or a cell penetrating peptide at the 3' end.

In an embodiment, the antisense strand may include one or more chemical modifications selected from: modification of 2 to 7 nucleotide bonds adjacent to the 3' end or the 5' end with phosphorothioate, boranophosphate, or methyl phosphonate; substitution of an -OH group at the 2' carbon position of the sugar structure of at least one nucleotide with -CH₃ (methyl), -OCH₃ (methoxy), -NH₂, -F, -O-2-methoxyethyl-O-propyl, - O-2-methyl methylthioethyl, -O-3-aminopropyl, or-O-3-dimethylaminopropyl; and binding with a phosphate group, E-vinylphosphonate, or a cell penetrating peptide at the 5' end.

In another embodiment, the RNAi agent may include at least one modification selected from the group consisting of: modification of 2 to 7 nucleotide bonds adjacent to the 3' end or the 5' end in a sense or an antisense strand to phosphorothioate; modification of an -OH group at the 2' carbon position of the sugar structure of at least one nucleotide in a sense strand or an antisense strand to -OCH₃ (methoxy), or -F; binding to an N-acetylgalactosamine (GalNAc) derivative at the 3' end of the sense strand; and binding with a phosphate group, or E-vinylphosphonate at the 5' end of an antisense strand.

In an embodiment, the sense strand may be a sequence selected from the antisense strand sequences listed in Table 2, Table 15, Table 16, and Table 17, and the antisense strand may include a sequence selected from the sense strand sequences listed in Table 2, Table 15, Table 16, and Table 17.

In an embodiment, the sense strand may be any one selected from the group consisting of (A) to (O) in the table below, and the antisense strand may be any one selected from the group consisting of (a) to (q) in the table below.

| | **Sequence (5' -> 3' )** |
|---|---|
| (A) | mG*fC*mGfCfAfGmCfUmCfUmGfGmAfUmCfU-GalNAc |
| (B) | mC*fC*mAfGfAfUmUfGmCfUmUfAmCfUmCfA-GalNAc |
| (C) | mC*fG*mAfAfAfGmUfUmAfUmAfUmGfGmAfA-GalNAc |
| (D) | mA*fC*mUfAfCfUmGfAmAfAmAfCmCfUmUfA-GalNAc |
| (E) | mG*fC*mAfUfAfUmGfUmCfAmGfUmUfGmUfA-GalNAc |
| (F) | mU*fU*mGfGfGfAmAfGmUfUmGfAmCfUmAfA-GalNAc |
| (G) | mC*fC*mAfUfUfUmUfGmUfCmCfUmUfUmGfA-GalNAc |
| (H) | mA*fC*mGfCfAfAmUfGmAfAmAfAmUfUmAfA-GalNAc |
| (I) | mA*fG*mAfUfCfUmGfAmUfGmAfAmGfUmAfA-GalNAc |
| (J) | mG*fC*mGfCfAfGmCfUmCfUmGfGmAfUmCfU-GalNAc |
| (K) | mC*fU*mAfGfAfGmAfAmGfAmAfAmGfUmUfA-GalNAc |
| (L) | mC*fC*mAfGfAfUmUmGmCmUmUmAmCfUmCfA-GalNAc |
| (M) | mC*fC*mAfGfGfUmGmGmCmCmUmAmCmUmCmA-GalNAc |
| (N) | mC*mC*mAfGmAfUmUmGmCmUmUmAmCmUmCmA-GalNAc |
| (O) | mC*fC*mCfUfGfAmCfUmCfUmCfAmGfUmGfA-GalNAc |
| (a) | P-mA*fG*mAmUmCfCmAfGfAmGmCmUmGfCmG*fC*mC*mA*mC |
| (b) | EVP-mA*fG*mAmUmCfCmAfGfAmGmCmUmGfCmG*fC*mC*mA*mC |
| (c) | P-mU*fG*mAmGmUmAfAmGfCmAmAmUmCfUmGfGmU*mC*mC*mU*mU |
| (d) | P-mU*fU*mCmCmAmUfAmUfAmAmCmUmUfUmCfGmU*mU*mC*mU*mG |
| (e) | P-mU*fA*mAmGmGmUfUmUfUmCmAmGmUfAmGfUmC*mU*mA*mU*mC |
| (f) | P-mU*fA*mCmAmAmCfUmGfAmCmAmUmAfUmGfCmU*mU*mU*mC*mC |
| (g) | P-mU*fU*mAmGmUmCfAmAfCmUmUmCmCfCmAfAmU*mA*mU*mA*mA |
| (h) | P-mU*fC*mAmAmAmGfGmAfCmAmAmAmAfUmGfGmC*mA*mA*mU*mA |
| (i) | P-mU*fU*mAmAmUmUfUmUfCmAmUmUmGfCmGfUmA*mC*mC*mU*mC |
| (j) | P-mU*fU*mAmCmUmUfCmAfUmCmAmGmAfUmCfUmU*mA*mG*mA*mG |
| (k) | P-mA*fG*mAmUmCmCfAmGfAmGmCmUmGfCmGfCmC*mA*mC*mU*mG |
| (l) | P-mU*fA*mAmCmUmUfUmCfUmUmCmUmCfUmAfGmC*mC*mU*mG*mG |
| (m) | EVP-mU*fG*mAmGmUmAfAmGfCmAmAmUmCfUmGfGmU*mC*mC*mU*mU |
| (n) | EVP-mU*fG*mAmGmUmAmAmGmCmAmAmUmCfUmGfGmU*mC*mC*mU*mU |
| (o) | EVP-mU*fG*mAmGmUmAfAmGfCmAmAmUmCfUmG*fG*mU*mC*mC*mU*mU |
| (p) | EVP-mU*fG*mAmGmUmAmAmGmCmAmAmUmCfUmG*fG*mU*mC*mC*mU*mU |
| (q) | P-mU*fC*mAmCmUmGfAmGfAmGmUmCmAfGmGfGmU*mG*mU*mC*mA |

In the above sequence, * means a phosphorothioated bond, m means 2'-O-methyl, f means 2'-fluoro, GalNAc means a trivalent GalNAc derivative of Formula 1, P means 5' -phosphate group, and EVP means 5'-E-vinylphosphonate bond.

Specifically, the sense strand and antisense strand of the RNAi agent may be a sequence of the following sense strands and antisense strands selected from the above table.

| **NO.** | **Sense strand** | **Antisense strand** |
|---|---|---|
| 1 | (A) | (a) or (b) |
| 2 | (B) | (c), (m), (n), (o), or (p) |
| 3 | (C) | (d) |
| 4 | (D) | (e) |
| 5 | (E) | (f) |
| 6 | (F) | (g) |
| 7 | (G) | (h) |
| 8 | (H) | (i) |
| 9 | (I) | (j) |
| 10 | (J) | (k) |
| 11 | (K) | (l) |
| 12 | (L) | (m), (n), (o), or (p) |
| 13 | (M) | (m), (n), (o), or (p) |
| 14 | (N) | (m), (n), (o), or (p) |
| 15 | (O) | (q) |

Another aspect provides a pharmaceutical composition for preventing or treating a liver disease including the RNAi agent as an active ingredient.

Still another aspect provides a use of the RNAi agent for manufacturing a pharmaceutical product for preventing or treating a liver disease.

Since the pharmaceutical composition contains or uses the above-described RNAi agent as it is, descriptions thereof are omitted in order to avoid excessive complexity of the present specification.

### liver disease

The pharmaceutical composition may be utilized as an active ingredient of a pharmaceutical composition for preventing or a treating liver disease by inhibiting expression of a MARC1 gene.

The liver disease includes fatty liver, liver fibrosis, or cirrhosis. Here, "fatty liver" refers to an occurrence of phenomenon in which triglycerides, which do not exist in normal cells, are abnormally deposited in hepatocytes. About 5 % of normal liver is composed of fatty tissue, and triglycerides, fatty acids, phospholipids, cholesterol and cholesterol esters are main components of fat, however, once fatty liver occurs, most of the components are replaced with triglycerides, and when an amount of triglycerides is more than 5 % of the liver weight, fatty liver is diagnosed. The fatty liver may be "non-alcoholic fatty liver disease (NAFLD)", and the "non-alcoholic fatty liver disease (NAFLD)" is a disease in which triglycerides are accumulated in the liver regardless of drinking and may be defined as a case in which fatty acids are accumulated in the parenchymal cells of the liver by 5% or more in the form of triglycerides. The non-alcoholic fatty liver disease may be simple steatosis or non-alcoholic steatohepatitis (NASH). Non-alcoholic fatty liver disease is pathologically classified into simple steatosis and non-alcoholic steatohepatitis (NASH) with inflammation, and NASH may be said to be a severe form of NAFLD. Steatosis with severe fat accumulation progresses to inflammation, that is, steatohepatitis, and when left untreated for a long time, steatohepatitis may lead to serious liver diseases such as hepatitis, liver fibrosis, and cirrhosis. Incidence of simple steatosis rises significantly from 10 % to 15 % in people with normal weight to 80 % in overweight people, therefore, in order to effectively treat nonalcoholic steatohepatitis, it may be important to contain the progression to the next step by effectively reducing accumulation of triglycerides in the liver, and by improving inflammation of steatohepatitis while inhibiting the progression of nonalcoholic steatosis to steatohepatitis.

### Pharmaceutical composition

The term "effective ingredient", used herein, means an appropriate effective amount of an ingredient that brings about a beneficial or desirable clinical or biochemical outcome. Specifically, the term may refer to an agent, an active agent, or an RNAi agent of an effective amount.

The effective amount may be administered one or more times, and unlimitedly may be an appropriate amount for preventing a disease, alleviating symptoms, reducing the extent of the disease, stabilizing (i.e., not exacerbating) the disease state, delaying or reducing the rate of disease progression, or improving or temporarily alleviating and ameliorating (partially or fully) the disease state.

The term "prevention", used herein, refers to any action that blocks an occurrence of a disease in advance, suppresses a disease, or delays progression thereof. For example, the term refers to preventing the liver disease or occurrence of characteristics thereof, interrupting the occurrence, or defending or protecting from the liver disease or occurrence of the characteristics thereof.

The term "treatment", used herein, refers to both therapeutic treatment and preventive or prophylactic measures. In addition, it refers to any action in which the symptoms of a disease are improved or beneficially changed. For example, the term refers to preventing, reducing or improving the liver disease or characteristics thereof, or delaying (attenuating) progression of the liver disease or characteristics thereof in a subject.

The term "effective amount", used herein, refers to a generally accepted meaning in the art. The term generally refers to an amount of a molecule, compound, or construct that elicits an intended biological response (e.g., a beneficial response) in a cell, tissue, a system, an animal, or a human, sought by researchers, veterinarians, physicians, or other clinicians, etc. Specifically, a "therapeutically effective amount" refers to an amount of a molecule, compound, or construct that is capable of eliciting a desirable medical response to the extent that a particular clinical treatment may be considered effective, due to, for example, a therapeutically relevant change in a measurable parameter associated with the disease or disorder. The therapeutically effective amount of a drug for treatment of a disease or a disorder may be an amount necessary to cause a therapeutically relevant change in the parameter.

A method of administering the pharmaceutical composition may be determined by a person skilled in the art based on the symptoms of the patient and the severity of the disease. In addition, the pharmaceutical composition may be formulated in various forms such as powders, tablets, capsules, solutions, injections, ointments, syrups, etc., and may be provided in unit-dose or multi-dose containers, for example, sealed ampoules and bottles.

The pharmaceutical composition of the present disclosure may be administered orally or parenterally. Routes of administration of the composition according to the present disclosure may be, for example, oral, intravenous, intramuscular, intraarterial, intramedullary, intrathecal, intracardiac, transdermal, subcutaneous, intraperitoneal, intestinal, sublingual, or topical, but are not limited thereto. Dosage of the composition according to the present disclosure varies depending on the patient's weight, age, sex, health status, diet, administration time, method, excretion rate, or severity of disease, etc. and may be readily determined by those of average skill in the art. In addition, the composition of the present disclosure may be formulated into a suitable formulation for clinical administration using known techniques.

Another aspect provides a method of preventing or treating liver disease, including administering the RNAi agent to a subject.

Since the method of treating liver disease contains or uses the above-described pharmaceutical composition as it is, descriptions thereof are omitted in order to avoid excessive complexity of the present specification.

The term "subject", used herein, refers to a subject in need of treatment for a disease, specifically, liver disease, and more specifically, the term may include all mammals such as a human or a non-human primate, a mouse, a dog, a cat, a horse, a cow, sheep, a pig, a goat, a camel, and an antelope.

Hereinafter, the present disclosure will be described in more detail through examples. However, these examples are intended to illustrate the present disclosure, and the scope of the present disclosure is not limited to these examples.

### Example 1: Screening and evaluating of therapeutic efficacy of nucleic acid molecules for inducing RNAi

### 1-1. Design of 50 types of asiRNAs and evaluation of expression inhibitory effect on MARC1 mRNA.

In this example, in order to secure a double-stranded nucleic acid molecule that induces high-efficiency RNA interference targeting MARC1, after selecting target sequences for the MARC1 gene, MARC1 asymmetric siRNAs (asiRNAs) for the same were designed (sense strand (16-mer), antisense strand (19-mer)). Specifically, after obtaining MARC1 gene information through NCBI database search, nucleotide sequences showing a certain level of homology were selected, based on the nucleotide sequences having a common target with a mouse, in consideration of animal experiments, and a total of 50 asiRNAs were designed and the same were synthesized at 10 nmole scale by IDT Korea, Inc. Thereafter, the synthesized asiRNAs were annealed through an incubation process at 95 °C for 5 minutes and 37 °C for 25 minutes, and after 12 % polyacrylamide gel electrophoresis (PAGE), quality control (QC) was performed by using a ChemiDoc UV transilluminator (Biorad).

Sequence information of the MARC1 asiRNAs designed according to the above method is shown in Table 1 below.

**[Table 1]**

| **NO.** | **asiRNA name** | **Sequence(5' → 3')** | | **SEQ NO.** |
|---|---|---|---|---|
| #1 | asiMARC1-1 | sense | GCUCUGGAUCUACCCU | 1 |
| | | antisense | AGGGUAGAUCCAGAGCUGC | 2 |
| #2 | asiMARC1-2 | sense | CAGCUCUGGAUCUACC | 3 |
| | | antisense | GGUAGAUCCAGAGCUGCGC | 4 |
| #3 | asiMARC1-3 | sense | GCAGCUCUGGAUCUAC | 5 |
| | | antisense | GUAGAUCCAGAGCUGCGCC | 6 |
| #4 | asiMARC1-4 | sense | GCCAUGGGGCUGCGCA | 7 |
| | | antisense | UGCGCAGCCCCAUGGCCGU | 8 |
| #5 | asiMARC1-5 | sense | GGCCAUGGGGCUGCGC | 9 |
| | | antisense | GCGCAGCCCCAUGGCCGUG | 10 |
| #6 | asiMARC1-6 | sense | CGGCCAUGGGGCUGCG | 11 |
| | | antisense | CGCAGCCCCAUGGCCGUGC | 12 |
| #7 | asiMARC1-7 | sense | ACGGCCAUGGGGCUGC | 13 |
| | | antisense | GCAGCCCCAUGGCCGUGCA | 14 |
| #8 | asiMARC1-8 | sense | CACGGCCAUGGGGCUG | 15 |
| | | antisense | CAGCCCCAUGGCCGUGCAC | 16 |
| #9 | asiMARC1-9 | sense | GGACAGGUUUUGGCUU | 17 |
| | | antisense | AAGCCAAAACCUGUCCCGC | 18 |
| #10 | asiMARC1-10 | sense | GGGACAGGUUUUGGCU | 19 |
| | | antisense | AGCCAAAACCUGUCCCGCA | 20 |
| #11 | asiMARC1-11 | sense | CGGGACAGGUUUUGGC | 21 |
| | | antisense | GCCAAAACCUGUCCCGCAG | 22 |
| #12 | asiMARC1-12 | sense | AGCCUACACAAAGGAC | 23 |
| | | antisense | GUCCUUUGUGUAGGCUGCA | 24 |
| #13 | asiMARC1-13 | sense | GGUGCACUUCGAGCCU | 25 |
| | | antisense | AGGCUCGAAGUGCACCAGG | 26 |
| #14 | asiMARC1-14 | sense | CUGGUGCACUUCGAGC | 27 |
| | | antisense | GCUCGAAGUGCACCAGGCG | 28 |
| #15 | asiMARC1-15 | sense | CCUGGUGCACUUCGAG | 29 |
| | | antisense | CUCGAAGUGCACCAGGCGG | 30 |
| #16 | asiMARC1-16 | sense | GCCUGGUGCACUUCGA | 31 |
| | | antisense | UCGAAGUGCACCAGGCGGU | 32 |
| #17 | asiMARC1-17 | sense | CGCCUGGUGCACUUCG | 33 |
| | | antisense | CGAAGUGCACCAGGCGGUA | 34 |
| #18 | asiMARC1-18 | sense | CCGCCUGGUGCACUUC | 35 |
| | | antisense | GAAGUGCACCAGGCGGUAG | 36 |
| #19 | asiMARC1-19 | sense | ACCGCCUGGUGCACUU | 37 |
| | | antisense | AAGUGCACCAGGCGGUAGG | 38 |
| #20 | asiMARC1-20 | sense | UACCGCCUGGUGCACU | 39 |
| | | antisense | AGUGCACCAGGCGGUAGGG | 40 |
| #21 | asiMARC1-21 | sense | CUACCGCCUGGUGCAC | 41 |
| | | antisense | GUGCACCAGGCGGUAGGGC | 42 |
| #22 | asiMARC1-22 | sense | GACGUGGAACUGAAAA | 43 |
| | | antisense | UUUUCAGUUCCACGUCACC | 44 |
| #23 | asiMARC1-23 | sense | UGACGUGGAACUGAAA | 45 |
| | | antisense | UUUCAGUUCCACGUCACCA | 46 |
| #24 | asiMARC1-24 | sense | GGAAACACUGAAGAGU | 47 |
| | | antisense | ACUCUUCAGUGUUUCCAGC | 48 |
| #25 | asiMARC1-25 | sense | UGGAAACACUGAAGAG | 49 |
| | | antisense | CUCUUCAGUGUUUCCAGCG | 50 |
| #26 | asiMARC1-26 | sense | CUGGAAACACUGAAGA | 51 |
| | | antisense | UCUUCAGUGUUUCCAGCGG | 52 |
| #27 | asiMARC1-27 | sense | GCUGGAAACACUGAAG | 53 |
| | | antisense | CUUCAGUGUUUCCAGCGGU | 54 |
| #28 | asiMARC1-28 | sense | CGCUGGAAACACUGAA | 55 |
| | | antisense | UUCAGUGUUUCCAGCGGUU | 56 |
| #29 | asiMARC1-29 | sense | CCGCUGGAAACACUGA | 57 |
| | | antisense | UCAGUGUUUCCAGCGGUUC | 58 |
| #30 | asiMARC1-30 | sense | CGCAGCUCUGGAUCUA | 59 |
| | | antisense | UAGAUCCAGAGCUGCGCCA | 60 |
| #31 | asiMARC1-31 | sense | GCGCAGCUCUGGAUCU | 61 |
| | | antisense | AGAUCCAGAGCUGCGCCAC | 62 |
| #32 | asiMARC1-32 | sense | GGCGCAGCUCUGGAUC | 63 |
| | | antisense | GAUCCAGAGCUGCGCCACU | 64 |
| #33 | asiMARC1-33 | sense | GCCGGUGAGCGAGGCG | 65 |
| | | antisense | CGCCUCGCUCACCGGCACC | 66 |
| #34 | asiMARC1-34 | sense | CCUGGUCCUGAUUUCC | 67 |
| | | antisense | GGAAAUCAGGACCAGGCGA | 68 |
| #35 | asiMARC1-35 | sense | GCCUGGUCCUGAUUUC | 69 |
| | | antisense | GAAAUCAGGACCAGGCGAG | 70 |
| #36 | asiMARC1-36 | sense | CGCCUGGUCCUGAUUU | 71 |
| | | antisense | AAAUCAGGACCAGGCGAGG | 72 |
| #37 | asiMARC1-37 | sense | UGACUCUCAGUGCAGC | 73 |
| | | antisense | GCUGCACUGAGAGUCAGGG | 74 |
| #38 | asiMARC1-38 | sense | CUGACUCUCAGUGCAG | 75 |
| | | antisense | CUGCACUGAGAGUCAGGGU | 76 |
| #39 | asiMARC1-39 | sense | CCUGACUCUCAGUGCA | 77 |
| | | antisense | UGCACUGAGAGUCAGGGUG | 78 |
| #40 | asiMARC1-40 | sense | CCCUGACUCUCAGUGC | 79 |
| | | antisense | GCACUGAGAGUCAGGGUGU | 80 |
| #41 | asiMARC1-41 | sense | ACCCUGACUCUCAGUG | 81 |
| | | antisense | CACUGAGAGUCAGGGUGUC | 82 |
| #42 | asiMARC1-42 | sense | GCACGGCCUGGAGAUA | 83 |
| | | antisense | UAUCUCCAGGCCGUGCACU | 84 |
| #43 | asiMARC1-43 | sense | GAUCCUUUCUGAGGCG | 85 |
| | | antisense | CGCCUCAGAAAGGAUCAAG | 86 |
| #44 | asiMARC1-44 | sense | UGAUCCUUUCUGAGGC | 87 |
| | | antisense | GCCUCAGAAAGGAUCAAGA | 88 |
| #45 | asiMARC1-45 | sense | UCUCAACUCCAGGCUA | 89 |
| | | antisense | UAGCCUGGAGUUGAGAUCC | 90 |
| #46 | asiMARC1-46 | sense | AUCUCAACUCCAGGCU | 91 |
| | | antisense | AGCCUGGAGUUGAGAUCCG | 92 |
| #47 | asiMARC1-47 | sense | GGAUCUCAACUCCAGG | 93 |
| | | antisense | CCUGGAGUUGAGAUCCGCC | 94 |
| #48 | asiMARC1-48 | sense | CGGAUCUCAACUCCAG | 95 |
| | | antisense | CUGGAGUUGAGAUCCGCCA | 96 |
| #49 | asiMARC1-49 | sense | GCGGAUCUCAACUCCA | 97 |
| | | antisense | UGGAGUUGAGAUCCGCCAG | 98 |
| #50 | asiMARC1-50 | sense | AGGGUGAUGGCUUGUU | 99 |
| | | antisense | AACAAGCCAUCACCCUUUU | 100 |

Then, in order to confirm expression inhibitory efficiency at an mRNA level, the MARC1 asiRNAs were transfected into Huh7 cells, and quantitative reverse transcription PCR (qRT-PCR) was performed to measure levels of MARC1 mRNA expression. Specifically, the Huh7 cells were seeded at 8×10³ cells/well in a 96 well plate, and MARC1 asiRNA (10 nM, OliX Inc.) and RNAiMax (2 µl/ml, Invitrogen Inc. 13778150) were added thereto, and transfection was performed according to the protocol provided by Invitrogen. Then, total RNA was extracted by using a RNeasy Plus Mini Kit (Qiagen, 74136), and cDNA was synthesized through the reverse transcription process using the mRNA contained therein as a template. Thereafter, a PCR mixture was prepared using TB Green (Takara, RR820A), and quantitative PCR was performed in a StepOne Real-Time PCR system according to the manufacturer's instructions. On the other hand, in this example, a group (NT) in which only a transfection reagent was used was used as a control group.

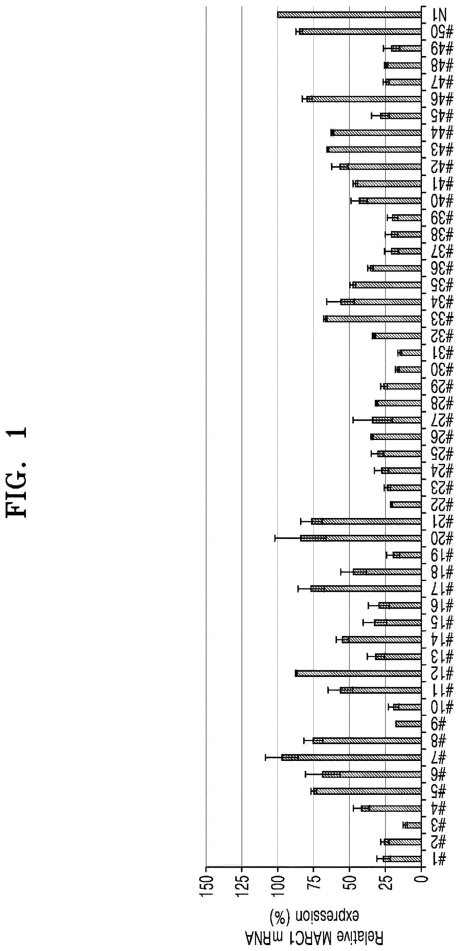

As a result, as shown in FIG. 1, it was confirmed that treatment of 50 types of MARC1 asiRNAs had expression inhibitory effect on MARC1 mRNA.

### 1-2. Design of 23 types of GalNAc-asiRNAs and evaluation of expression inhibitory effect on MARC1 mRNA.

In this example, 23 types of MARC1 asiRNAs among the MARC1 asiRNAs prepared in Example 1 were subjected to designing of asymmetric siRNAs into which GalNAc ligands and chemical modifications are introduced. Specifically, MARC1 GalNAc-asiRNAs were prepared by introducing various chemical modifications (2'OMe, PS, Fluoro, etc.) and binding a derivative labeled "trivalent GalNAc" to the 3' end of the sense strand.

**[Table 2]**

| **GalNAc-asiRNA name** | **Sequence(5' → 3')** | |
|---|---|---|
| OLX-001-1 | sense | mG*fC*mUfCfUfGmGfAmUfCmUfAmCfCmCfU-GalNAc |
| | antisense | P-mA*fG*mGmGmUfAmGfAfUmCmCmAmGfAmG*fC*mU*mG*mC |
| OLX-002-1 | sense | m C*fA*m GfCfUfC m UfG m GfAm UfCm UfAm CfC-Gal NAc |
| | antisense | P-mG*fG*mUmAmGfAmUfCfCmAmGmAmGfCmU*fG*mC*mG*mC |
| OLX-003-1 | sense | mG*fC*mAfGfCfUmCfUmGfGmAfUmCfUmAfC-GalNAc |
| | antisense | P-mG*fU*mAmGmAfUmCfCfAmGmAmGmCfUmG*fC*mG*mC*mC |
| OLX-009-1 | sense | mG*fG*mAfCfAfGmGfUmUfUmUfGmGfCmUfU-GalNAc |
| | antisense | P-mA*fA*mGmCmCfAmAfAfAmCmCmUmGfUmC*fC*mC*mG*mC |
| OLX-010-1 | sense | mG*fG*mGfAfCfAmGfGmUfUmUfUmGfGmCfU-GalNAc |
| | antisense | P-mA*fG*mCmCmAfAmAfAfCmCmUmGmUfCmC*fC*mG*mC*mA |
| OLX-016-1 | sense | mG*fC*mCfUfGfGmUfGmCfAmCfUmUfCmGfA-GalNAc |
| | antisense | P-mU*fC*mGmAmAfGmUfGfCmAmCmCmAfGmG*fC*mG*mG*mU |
| OLX-019-1 | sense | mA*fC*mCfGfCfCm UfGmGfUm GfCmAfCm UfU-Gal NAc |
| | antisense | P-mA*fA*mGmUmGfCmAfCfCmAmGmGmCfGmG*fU*mA*mG*mG |
| OLX-022-1 | sense | mG*fA*mCfGfUfGmGfAmAfCmUfGmAfAmAfA-GalNAc |
| | antisense | P-m U*fU*m U m U mCfAm Gf UfU m CmCmAm CfGm U*fC*mA*mC*mC |
| OLX-023-1 | sense | m U*fG*mAfCfGfU m GfG mAfAm CfU mGfAmAfA-GalNAc |
| | antisense | P-mU*fU*mUmCmAfGmUfUfCmCmAmCmGfUmC*fA*mC*mC*mA |
| OLX-024-1 | sense | mG*fG*mAfAfAfCmAfCmUfGmAfAmGfAmGfU-GalNAc |
| | antisense | P-mA*fC*mUmCmUfUmCfAfGmUmGmUmUfUmC*fC*mA*mG*mC |
| OLX-027-1 | sense | m G*fC*m UfGfGfAmAfAm CfAm CfU m GfAmAfG-Gal NAc |
| | antisense | P-mC*fU*mUmCmAfGmUfGfUmUmUmCmCfAmG*fC*mG*mG*mU |
| OLX-029-1 | sense | mC*fC*mGfCfUfG m GfAmAfAm CfAm CfU mGfA-Gal NAc |
| | antisense | P-m U*fC*mAm Gm UfGm UfUfU mCmCmAm GfCm G*fG*m U*m U*mC |
| OLX-030-4 | sense | mC*fG*mCfAmGfCmUfCmUfGmGmAmUfCmUfA-GalNAc |
| | antisense | P-mU*fA*mGfAmUfCmCfAmGfAmGfCmUfGmC*fG*mC*fC*mA |
| OLX-030-5 | sense | mC*fG*mCfAfGfCmUfCmUfGmGfAmUfCmUfA-GalNAc |
| | antisense | P-mU*fA*mGmAmUfCmCfAfGmAmGmCmUfGmC*fG*mC*mC*mA |
| OLX-031-1 | sense | mG*fC*mGfCfAfGmCfUmCfUmGfGmAfUmCfU-GalNAc |
| | antisense | P-mA*fG*mAmUmCfCmAfGfAmGmCmUmGfCmG*fC*mC*mA*mC |
| OLX-037-1 | sense | mU*fG*mAfCfUfCmUfCmAfGmUfGmCfAmGfC-GalNAc |
| | antisense | P-mG*fC*mUmGmCfAmCfUfGmAmGmAmGfUmC*fA*mG*mG*mG |
| OLX-038-1 | sense | mC*fU*mGfAfCfUmCfUmCfAmGfUmGfCmAfG-GalNAc |
| | antisense | P-mC*fU*mGmCmAfCmUfGfAmGmAmGmUfCmA*fG*mG*mG*mU |
| OLX-039-1 | sense | m C*fC*m UfGfAfCm UfCm UfCmAfGm UfGm CfA-Gal NAc |
| | antisense | P-mU*fG*mCmAmCfUmGfAfGmAmGmUmCfAmG*fG*mG*mU*mG |
| OLX-045-2 | sense | mU*fC*mUfCfAfAmCfUmCfCmAmGmGfCmUfA-GalNAc |
| | antisense | P-mU*fA*mGfCmCfUmGfGmAfGmUfUmGfAmG*fA*mU*fC*mC |
| OLX-045-5 | sense | mU*fC*mUfCfAfAm CfU m CfCmAfGm GfCm UfA-Gal NAc |
| | antisense | P-mU*fA*mGmCmCfUmGfGfAmGmUmUmGfAmG*fA*mU*mC*mC |
| OLX-047-1 | sense | m G*fG*mAfUfCfU m CfAmAfCm UfC mCfAm GfG-Gal NAc |
| | antisense | P-mC*fC*m U m GmGfAm GfUfU mGmAm GmAfUm C*fC*m G*m C*m C |
| OLX-048-1 | sense | mC*fG*mGfAfUfCmUfCmAfAmCfUmCfCmAfG-GaINAc |
| | antisense | P-mC*fU*mGmGmAfGmUfUfGmAmGmAmUfCmC*fG*mC*mC*mA |
| OLX-049-1 | sense | mG*fC*mGfGfAfUmCfUmCfAmAfCmUfCmCfA-GalNAc |
| | antisense | P-mU*fG*mGmAmGfUmUfGfAmGmAmUmCfCmG*fC*mC*mA*mG |

Specifically, chemical modifications denoted by "*", "m", "f", "P", and "GalNAc" in Table 2 above are as shown in Table 3 below.

**[Table 3]**

| **Notation** | **Chemical modification** |
|---|---|
| * | Phosphorothioate bond |
| m | 2'-O-methyl |
| f | 2'-fluoro |
| P | 5'-phosphate group |
| "GalNAc" | Trivalent GalNAc derivative of Formula 1 |

Specifically, in Table 3, "*" means a form in which an existing phosphodiester bond is substituted by a phosphorothioate bond, and "m" means a form in which an existing 2'-OH is substituted with 2'-O-methyl. In addition, "f" means, for example, in case of fG, a form in which 2'-OH of existing guanine (G) is substituted with fluorine, and "P" means a form in which a phosphate group is bonded to the 5'-end (a phosphate group is bonded to an oxygen bonded to the 5th carbon in the 5' end base). In addition, "GalNAc" refers to a form in which a trivalent GalNAc derivative of Formula 1 below is bonded to the 3' end of a sense strand.

Meanwhile, the structure of the trivalent GalNAc derivative is as shown in Formula 1 below.

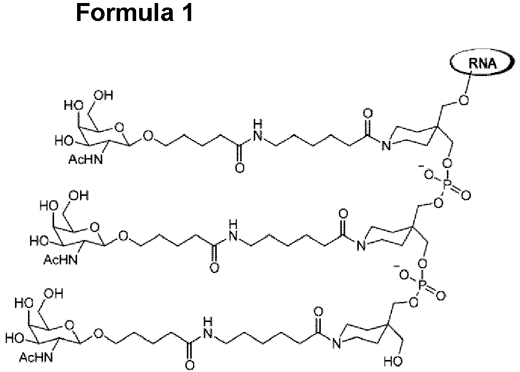

In order to identify expression inhibitory efficiency at an mRNA level, after transfection of the MARC1 GalNAc-asiRNA into mouse primary hepatocytes derived from a C57BL/6 mouse, qRT-PCR was performed to measure expression levels of MARC1 mRNA. Specifically, the primary hepatocytes were seeded at 7.5×10⁴ cells/well in a 24-well plate, and treated with MARC1 GalNAc-asiRNA (200 nM, OliX US, Inc.). 24 hours after the treatment, cell lysate was prepared by using a SuperPrep^{™} Cell lysis & RT kit for qPCR Kit II (TOYOBO, SCQ-401), and cDNA was synthesized through reverse transcription using the mRNA contained in the lysate as a template. Then, using the synthesized cDNA as a template, quantitative PCR was performed by using a THUNDERBIRD^{®} Probe qPCR Mix (TOYOBO, QPS-101), and Probes (Hs00224227_m1, Hs03928985_g1, Applied Biosystems). Then, expression levels of MARC1 mRNA were identified by using a CFX Connect Real-Time PCR System (Bio-Rad). On the other hand, in this example, a group not transfected (NT) was used as a control group, a group transfected according to the protocol provided by Invitrogen after OLX700A-001-8 (10 nM) and RNAiMAX (2 µl/ml, Invitrogen Inc. 13778150) were added, was used as a negative control group (NC), and a group transfected according to the protocol provided by Invitrogen after asiMARC1-30 (10nM) and RNAiMAX (2 µl/ml, Invitrogen Inc. 13778150) were added, was used as a positive control group (PC). The sequence information of CLX700A-001-8 is as shown in Table 4, and chemical modifications indicated by "*", "m", "f", "P", and "GalNAc" in Table 4 are as shown in Table 3 above.

**[Table 4]**

| **Name** | **Sequence(5' → 3')** | |
|---|---|---|
| OLX700A-001-8 | sense | mG*fC*mAfGfUfAmUfG mUfUmGfAmUfGmGfA-GalNAc |
| | antisense | P-mU*fC*mCfAmUfCmAfAmCfAmUmAmCfUmG*fC*mU*fU*mC |

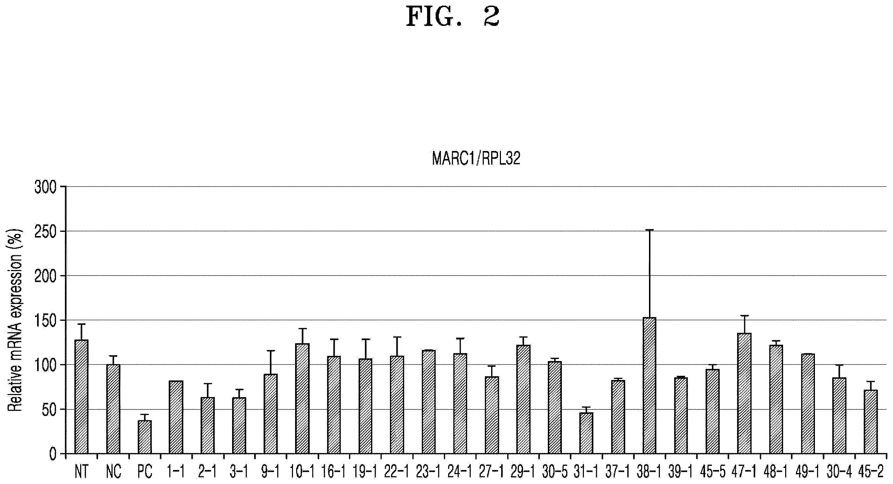

As a result, as shown in FIG. 2, it was confirmed that treatment of 23 types of MARC1 GalNAc-asiRNAs had expression inhibitory effect on MARC1 mRNA. Among the asiRNAs, expression inhibitory effects of OLX-002-1, OLX003-1, and OLX-031-1 were more excellent.

### 1-3. Evaluation of treatment efficacy using animal models

In this example, MARC1 GalNAc-asiRNAs, which were confirmed to have an expression inhibitory effect in Example 1-2, were administered to C57BL/6 mice (male, n=15), and then the therapeutic efficacy was evaluated. To this end, first, animal models were divided into a group provided with a normal diet (normal chow) and a group provided with a high fat diet (HFD, High Fat Diet (Research Diet, D12492)), and the groups were each classified into a group administered with 1X PBS (VC), a group administered with OLX-001-8 (NC), a group subcutaneously administered with OLX-003-1 (#3-1), and a group subcutaneously administered with OLX-031-1 (#31-1), according to the substance administered. In this example, specific classification of the animal model groups is as shown in Table 5 below.

**[Table 5]**

| **Group** | **Dose** | **Treatment** | | **Number** |
|---|---|---|---|---|
| 1 | 0 | Normal Chow | VC | 3 |
| 2 | 0 | HFD | VC | 4 |
| 3 | 9 mpk | | NC | 2 |
| 4 | 9 mpk | | OLX-003-1 | 2 |
| 5 | 9 mpk | | OLX-031-1 | 2 |

On the other hand, MARC1 GalNAc-asiRNAs were administered 16 weeks from the time when a high-fat diet was first provided, and 2 weeks after the first administration, MARC1 asiRNAs were administered again. Two weeks after the second administration was performed, the liver was extracted from the target mice, and liver tissue samples and sections thereof were obtained therefrom.

### (1) Confirmation of expression patterns of MARC1 and MARC2

The liver tissue samples obtained from the mice were evaluated for expression levels of MARC1 and MARC2 mRNA in the same manner as in Example 1-2.

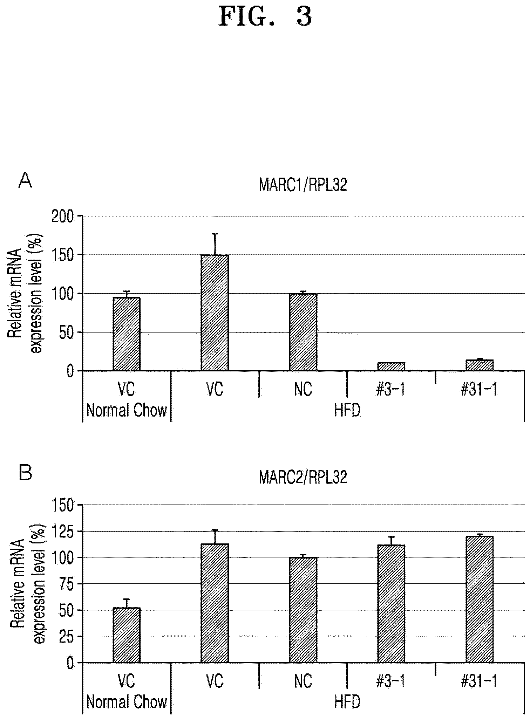

As a result, as shown in FIG. 3, in the groups (#3-1, #31-1) administered with OLX-003-1 or OLX-031-1 according to an embodiment, it was confirmed that expression of MARC2 mRNA was not affected, while the group showed suppressed expression of MARC1 mRNA, compared to other groups (VC, NC) provided with a high-fat diet.

### (2) Evaluation of appearance of liver

The livers obtained from the mice were visually observed to evaluate the change in the liver marginal region.

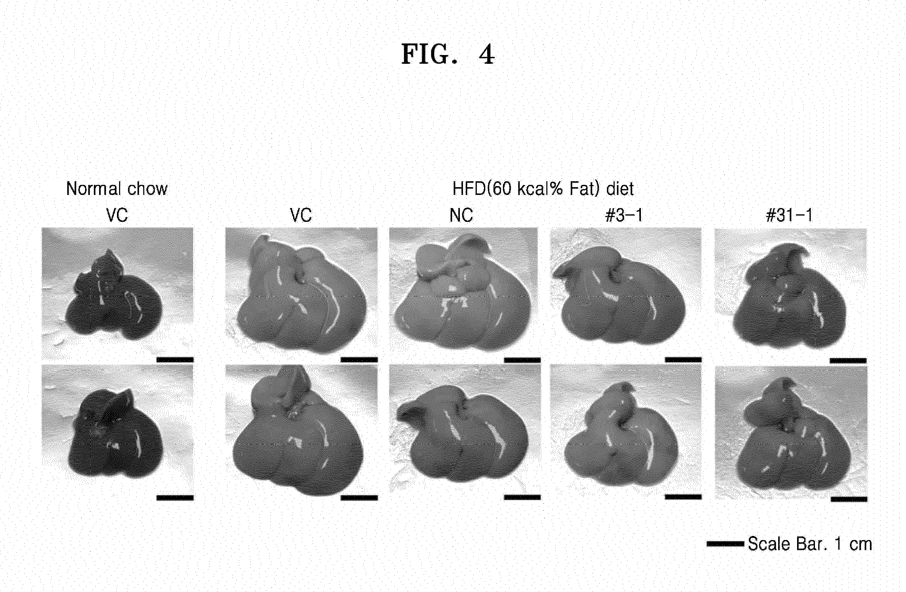

As a result, as shown in FIG. 4, it was confirmed that the groups (VC, NC) provided with a high-fat diet had edges of the liver, that is, marginal region of the liver that is not sharp, whereas the groups (#3-1, #31-1) administered with OLX-003-1 or OLX-031-1 according to an example exhibited morphological characteristics similar to those of the normal group.

### (3) Histopathological evaluation

Liver tissue samples obtained from the mice were fixed with a 10 % neutral buffered formalin (NBF) solution, and paraffin-embedded blocks were prepared, and then tissue sections having a thickness of 4 µm were prepared therefrom. Thereafter, the tissue sections were subjected to H&E staining using Mayer's hematoxylin reagent. In addition, Oil Red O staining was performed on the frozen sections with a thickness of 4 µm obtained by using optimal cutting temperature (OCT) embedding agent.

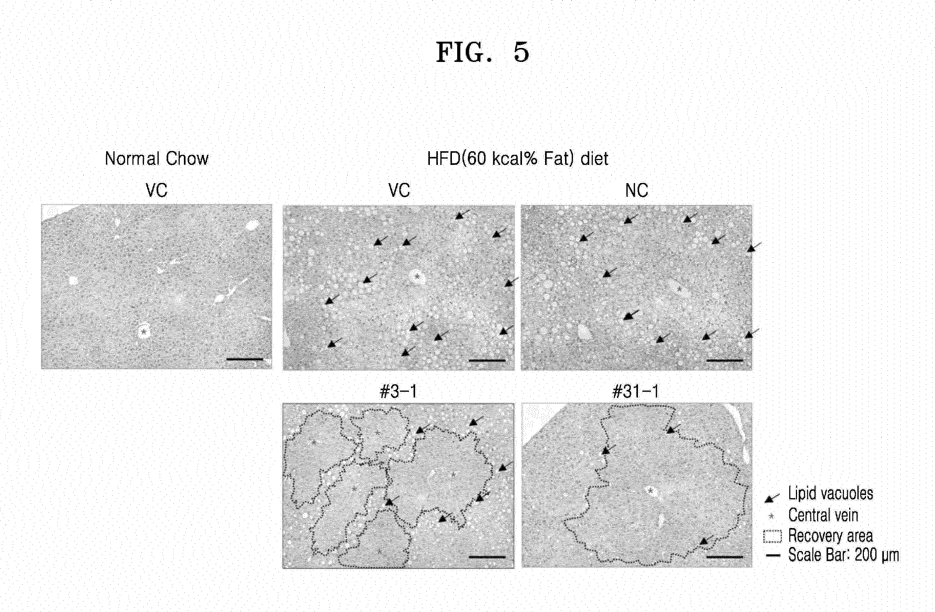

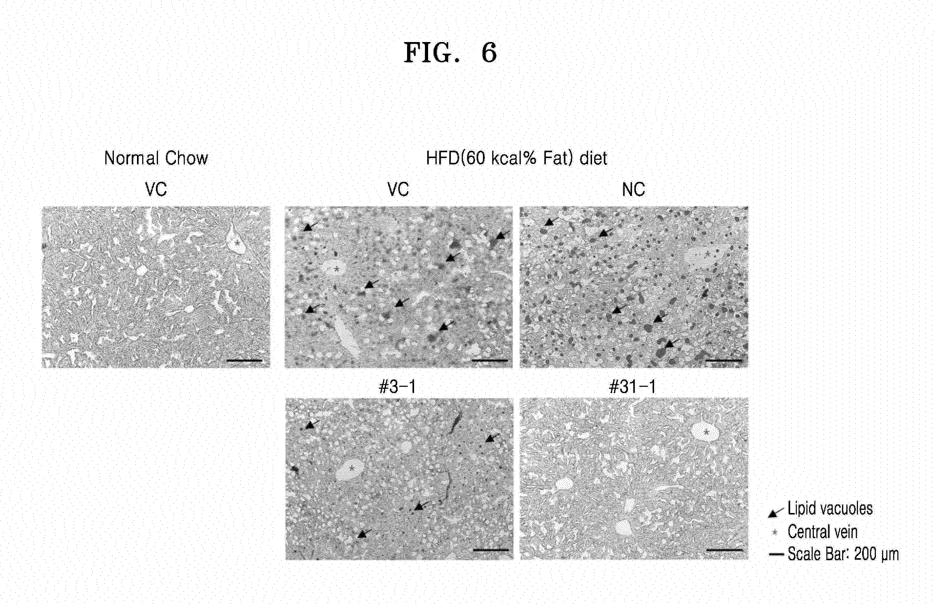

As a result, as shown in FIGS. 5 and 6 , in the groups (VC, NC) provided with a high-fat diet, a large number of lipid vacuoles formed in the liver parenchyma were observed, whereas in the groups (#3-1, #31-1) administered with OLX-003-1 or OLX-031-1 according to an embodiment, it was confirmed that formation of fatty vacuoles was significantly reduced.

### Example 2. Evaluation of treatment efficacy using animal models

In this example, MARC1 GalNAc-asiRNA of Table 6 below was subcutaneously administrated to C57BL/6 mice, with asiMARC1-031, which was confirmed to have an expression inhibitory effect in Example 1, as a template, and therapeutic efficacy thereof was evaluated.

**[Table 6]**

| **GalNAC-asiRNA name** | **Sequence(5' → 3')** | |
|---|---|---|
| OLX-031-1 | sense | mG*fC*mGfCfAfGmCfUmCfUmGfGmAfUmCfU-GalNAc |
| | antisense | P-mA*fG*mAmUmCfCmAfGfAmGmCmUmGfCmG*fC*mC*mA*mC |
| OLX-031-2 | sense | mG*fC*mGfCfAfGmCfU mCfU mGfGmAfU mCfU-Gal NAc |
| | antisense | EVP-mA*fG*mAmUmCfCmAfGfAmGmCmUmGfCmG*fC*mC*mA*mC |

Specifically, chemical modifications denoted by "*", "m", "f", "P", "GalNAc", and "EVP" in Table 6 are as shown in Table 7 below.

**[Table 7]**

| **Notation** | **Chemical modification** |
|---|---|
| * | Phosphorothioate bond |
| m | 2'-O-methyl |
| f | 2'-fluoro |
| P | 5'-phosphate group |
| "GalNAc" | Trivalent GalNAc derivative of Formula 1 |
| EVP | E-vinylphosphonate |

Specifically, in Table 7, "*", "m", "f", "P", and "GalNAc" are the same as described above. In addition, "EVP" refers to a form in which (E) vinylphosphonate is bonded to the 5'-end (forming a double bond including the 5th carbon in the 5'-end base), for example, EVP-mU refers to a form in which (E) vinyl phosphonate is bonded to the 5'-end in a form in which existing 2'-OH of uridine (U) is substituted with 2'-O-methyl.

### 2-1. Evaluation of therapeutic efficacy of OLX-031-1 in HFD-induced NASH mouse model

The animal models were divided into a group provided with a normal diet (Normal Chow) and a group provided with a high fat diet (HFD), and the groups were classified into a group administered with 1X PBS (VC), a group administered with OLX700A-001-8 (NC), and a group administered with OLX-031-1 (#31-1)., according to the substance administered. In this example, specific classification of animal model groups is as shown in Table 8 below.

**[Table 8]**

| **Group** | **Dose** | **Treatment** | | **Number** |
|---|---|---|---|---|
| 1 | 0 | Normal Chow | VC | 1 |
| 2 | 0 | HFD | VC | 3 |
| 3 | 10 mpk | | NC | 4 |
| 4 | 10 mpk | | OLX-031-1 | 5 |

On the other hand, OLX-031-1 was administered at 28 weeks from the time a high-fat diet was first provided, and OLX-031-1 was additionally administered 3 times from the first administration at intervals of 1 week. One week from the time a total of 4 administrations was completed, the livers were extracted from the target mice, and liver tissue samples and sections thereof were obtained therefrom.

### (1) Confirmation of expression patterns of MARC1 and MARC2

The liver tissue samples obtained from the mice were evaluated for expression levels of MARC1 and MARC2 mRNA in the same manner as in Example 1-2.

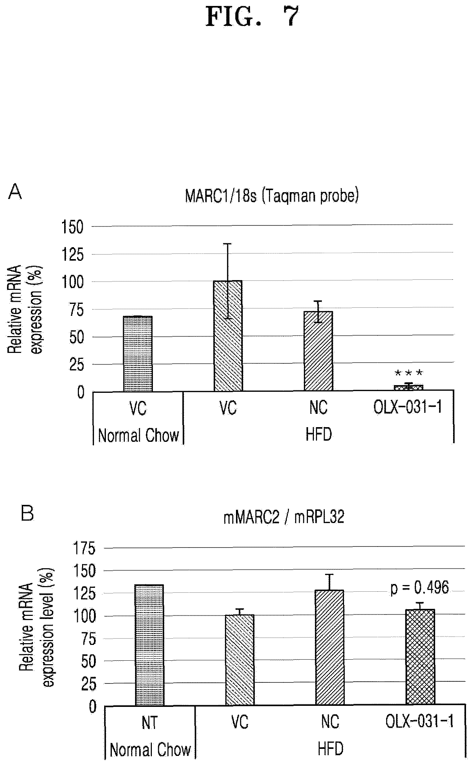

As a result, as shown in FIG. 7, in the group (#31-1) administered with OLX-031 - 1 according to an embodiment, it was confirmed that expression of MARC2 mRNA was not affected, while the group showed suppressed expression of MARC1 mRNA, compared to other groups (VC, NC) provided with a high-fat diet.

### (2) Evaluation of appearance of liver

The livers obtained from the mice were visually observed to evaluate the change in the liver marginal region.

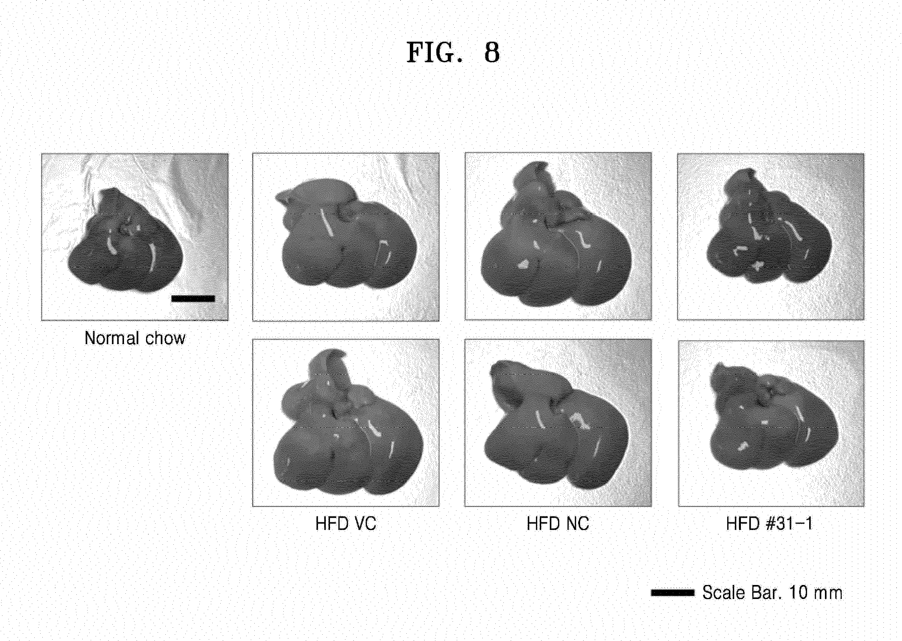

As a result, as shown in FIG. 8, it was confirmed that the groups (HFD VC, HFD NC) provided with a high-fat diet had edges of the liver, that is, a marginal region of the liver that is not sharp, whereas the group (#31-1) administered with OLX-31-1 according to an example exhibited morphological characteristics similar to those of the normal group.

### (3) Histopathological evaluation

Liver tissue samples obtained from the mice were fixed with a 10 % neutral buffered formalin (NBF) solution, and paraffin-embedded blocks were prepared, and then tissue sections having a thickness of 4 µm were prepared therefrom. Thereafter, the tissue sections were subjected to H&E staining and Picro Sirius Red staining using Mayer's hematoxylin reagent.

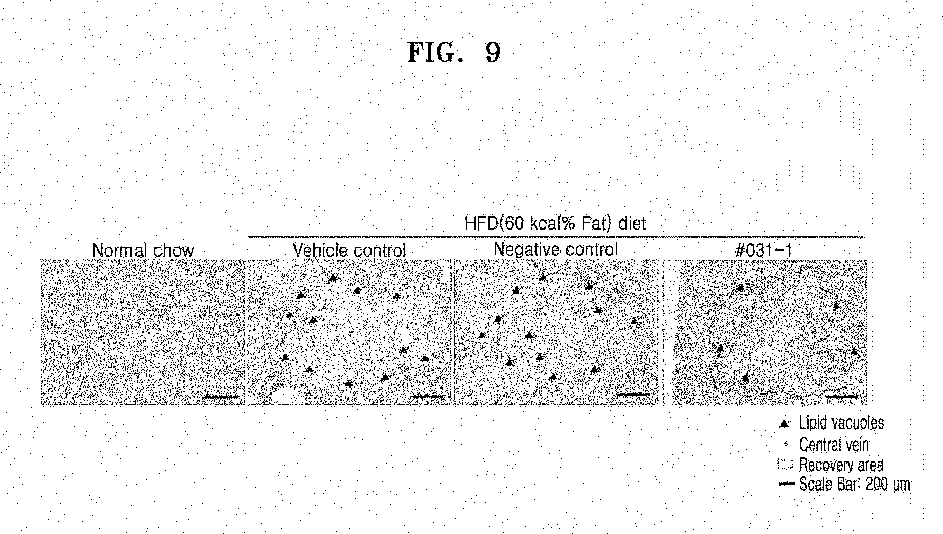

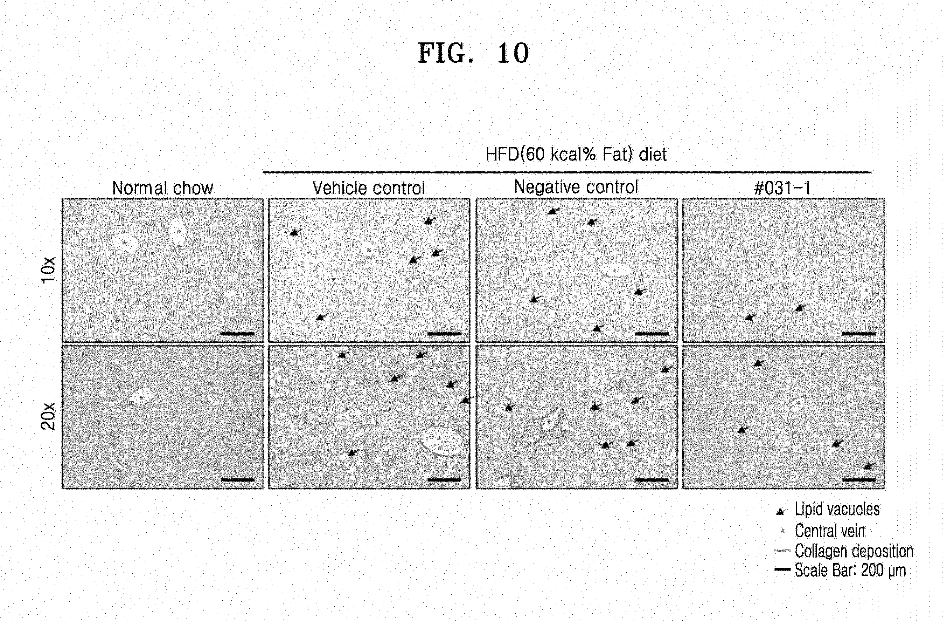

As a result, as shown in FIGS. 9 and 10, in the groups (VC, NC) provided with a high-fat diet, a large number of fatty vacuoles formed in the liver parenchyma and deposition of collagen were observed, whereas in the group administered with OLX-031-1 according to an embodiment, formation of these fatty vacuoles and collagen deposition was confirmed to be significantly reduced.

### (4) Evaluation of expression of fibrosis-related factors in liver tissue and triglyceride levels

The liver tissue samples obtained from the mice were evaluated for expression levels of liver fibrosis-related factors and levels of triglycerides. Specifically, total RNA was extracted from the liver tissue-derived cells using a Tri-RNA reagent (FAVORGEN, FATRR 001), and using the total RNA as a template, cDNA was synthesized through a reverse transcription process (High-capacity cDNA Reverse Transcription Kit (Applied Biosystems, 4368814)). Then, using the synthesized cDNA as a template, quantitative PCR was performed by using TB Green^{®} Premix Ex Taq^{™} (Takara, RR420A). Thereafter, expression levels of α-SMA and mCol1α1 mRNA were evaluated by applying the StepOne^{™} Real-Time PCR System (Applied Biosystems^{™}) and the expression level of RPL32, which is a house-keeping gene. In addition, liver tissue samples were homogenized in 5 % NP-40/UPW using a homogenizer (homogenizer, 100 mg/mL, 1:10 dilution). Thereafter, a level of triglyceride was measured in the homogenized sample using a Triglyceride Assay Kit (Abeam, ab65336).

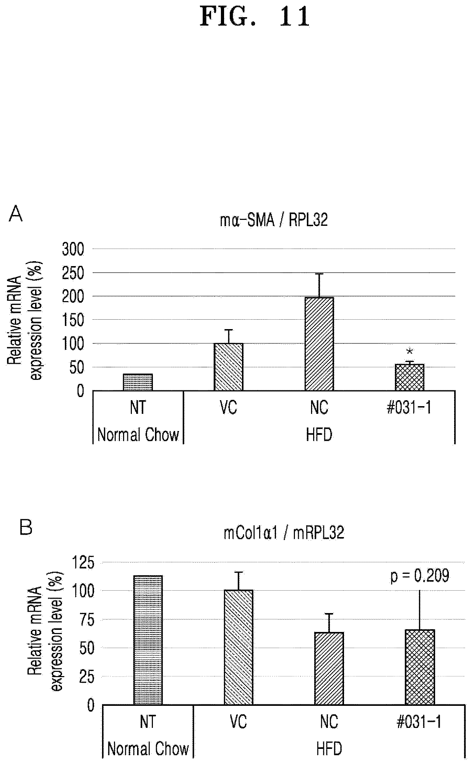

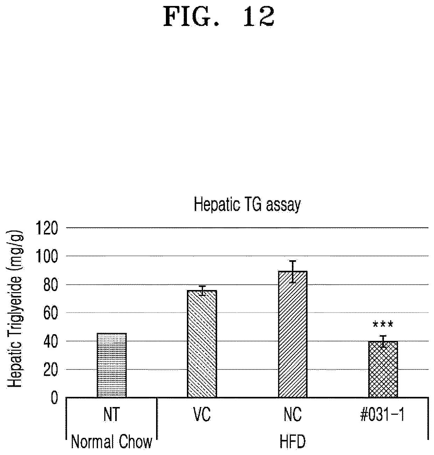

As a result, as shown in FIG. 11, in the groups (VC, NC) provided with a high-fat diet, the expression level of α-SMA mRNA, a factor related to liver fibrosis, increased, while in the group (#31-1) administered with OLX-031-1 according to an embodiment, it was confirmed that the increase of the expression of α-SMA and mCol1α1 mRNA was decreased. In addition, as shown in FIG. 12, it was confirmed that the level of triglycerides in liver tissue was also reduced by administration of OLX-031-1 according to an embodiment.

### (5) Serological evaluation

For the serum samples obtained from the mice, levels of aspartate aminotransferase (AST), and alanine aminotransferase (ALT), which are indicators of liver damage, and cholesterol (CHOL), triglycerides (TG), low-density lipoproteins (LDL), and high-density lipoprotein (HDL), which are lipid indicators, were identified.

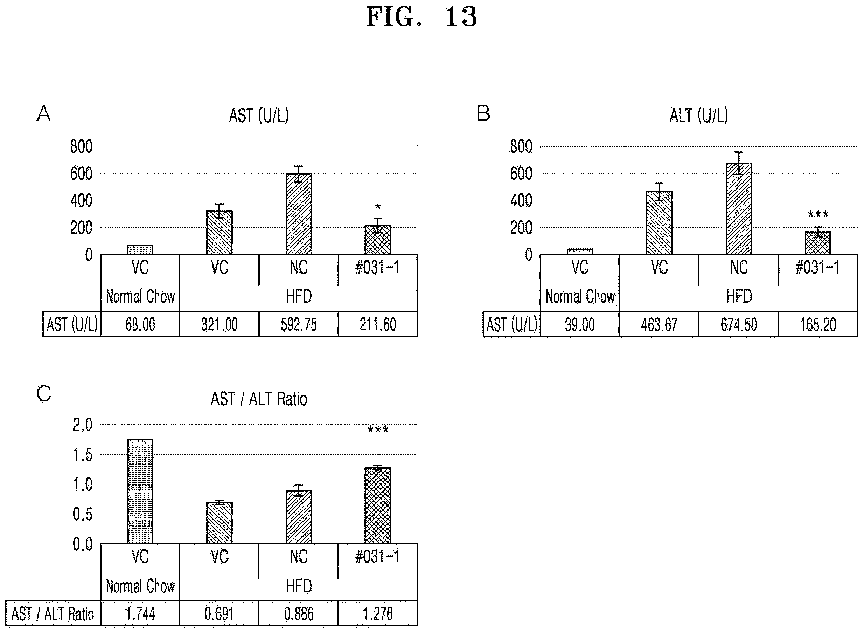

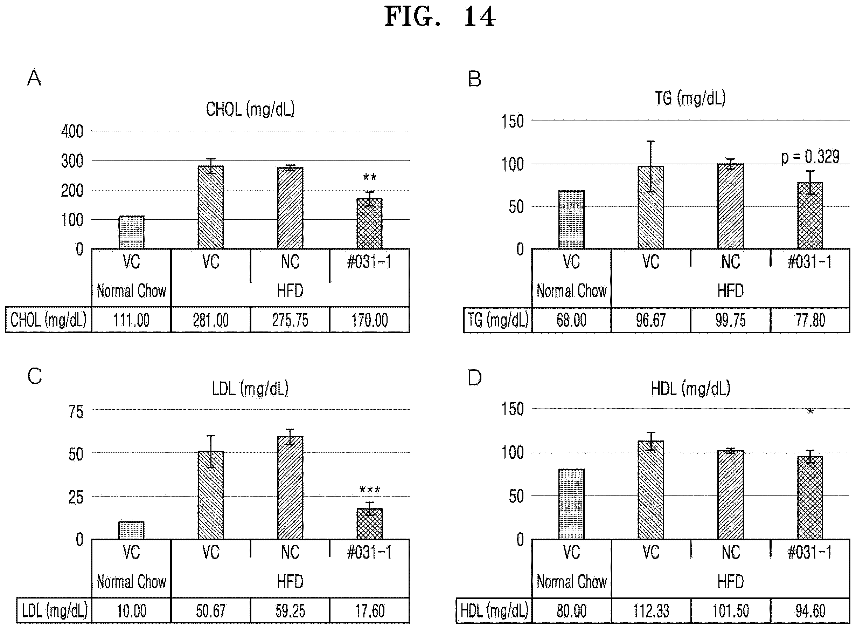

As a result, as shown in FIG. 13, in the groups (VC, NC) provided with a high-fat diet, AST and ALT levels were significantly increased, whereas in the group (#31-1) administered with OLX-031-1 according to an embodiment, it was confirmed that the increase in the AST and ALT levels was reduced. In addition, as shown in FIG. 14, it was confirmed that the level of lipid indicators was also decreased in the group (#31-1) administered with OLX-031-1 according to an embodiment.

### 2-2. Evaluation of therapeutic efficacy of OLX-031-2 in CDHFD-induced NASH mouse model

According to the type of diet and duration of feeding, the animal models were divided into a group provided with a normal diet for 12 weeks (NCD 12w), a group provided with a normal diet for 16 weeks (NCD 16w), a group provided with a choline-deficient high-fat diet for 12 weeks (CDHFD 12w), a group provided with a high-fat diet for 16 weeks (CDHFD 16w), and a group provided with a high-fat diet for 12 weeks and a normal diet for 4 weeks (CDHFD-NCD), and each of the groups were again divided into a group administered with 1X PBS (VC) and a group administered with OLX-031-2 (#31-2), according to the administered substance. In this example, specific classification of the animal model groups is as shown in Table 9 below.

**[Table 9]**

| **Group** | **Dose** | **Treatment** | | **Number** |
|---|---|---|---|---|
| 1 | 0 | Normal diet for 12 weeks (NCD 12W) | VC | 3 |
| 2 | 0 | Normal diet for 16 weeks (NCD 16W) | VC | 5 |
| 3 | 0 | High-fat diet for 12 weeks (CDHFD 12W) | VC | 3 |
| 4 | 0 | High-fat diet for 16 weeks (CDHFD 16W) | VC | 3 |
| 5 | 0 | High-fat diet for 12 weeks and normal diet for 4 weeks (CDHFD-NCD) | VC | 4 |
| 6 | 10 mpk | High-fat diet for 16 weeks (CDHFD 16W) | OLX-031-2 | 4 |
| 7 | 10 mpk | High-fat diet for 12 weeks and normal diet for 4 weeks (CDHFD-NCD) | OLX-031-2 | 4 |

On the other hand, OLX-031-2 was administered at 12 weeks from the time a high-fat diet was first provided, and OLX-031-2 was administered again 2 weeks after the first administration. Two weeks after the second administration was performed, the livers were extracted from the target mice, and liver tissue samples and sections thereof were obtained therefrom. On the other hand, in the animal model groups 1 and 3, mice were sacrificed at 12 weeks from the time a high-fat diet was first provided, and the samples were obtained.

### (1) Histopathological evaluation

H&E staining and Picro Sirius Red were performed on tissue sections in the same manner as in Example 2-1 (3), and changes such as a large number of fat vacuoles formed in the liver parenchyma and collagen deposition were confirmed.

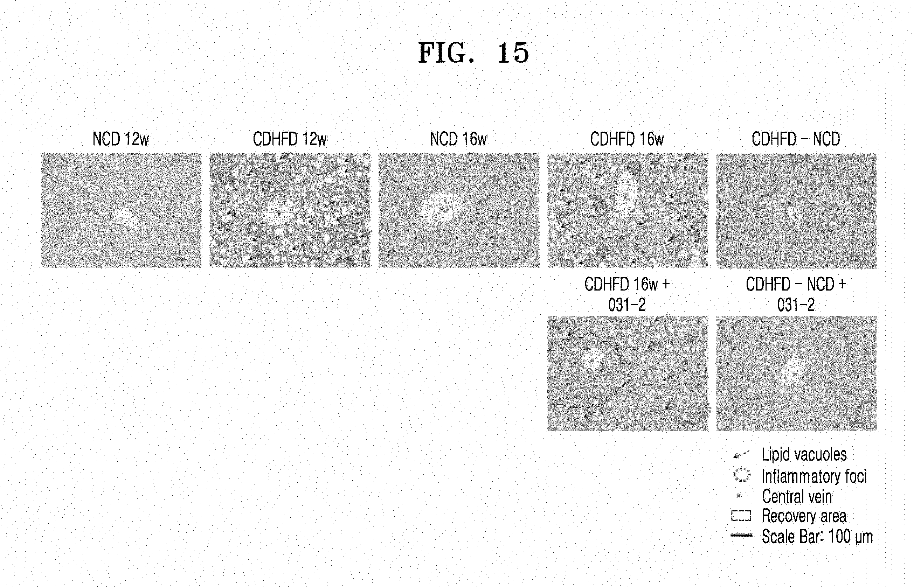

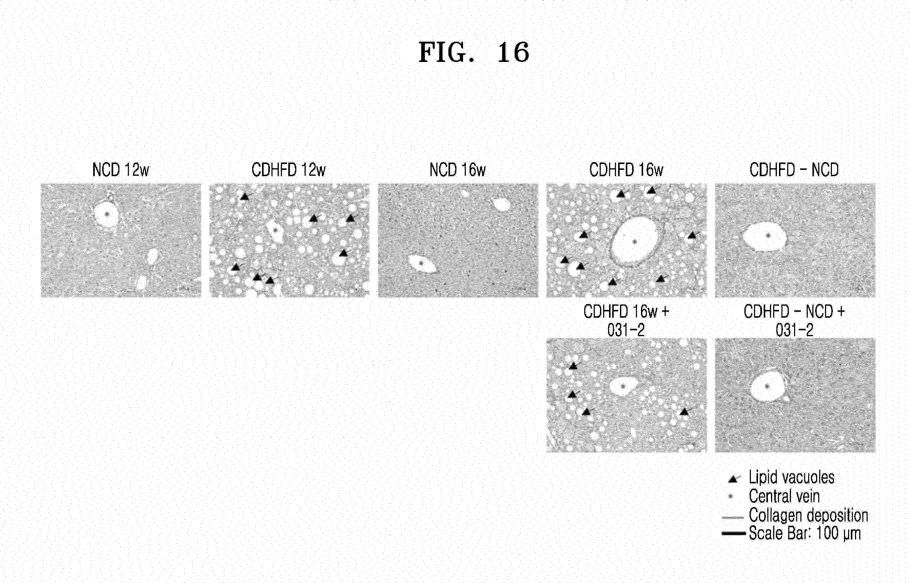

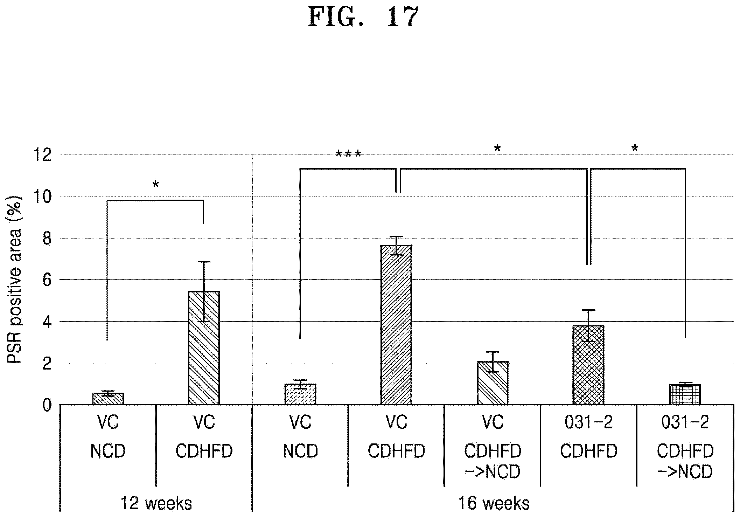

As a result, as shown in FIG. 15, in the groups provided with a high-fat diet (CDHFD 12w and CDHFD 16w), a large number of fatty vacuoles and inflammatory foci were observed in the liver parenchyma, whereas in the groups administered with OLX-031-2 according to an example (CDHFD 031-2 and CDHFD-NCD 031-2), it was confirmed that these fatty vacuoles and inflammatory foci were reduced. In addition, as shown in FIGS. 16 and 17, lipid components and collagen deposition were confirmed to be significantly reduced in the groups (CDHFD 031-2 and CDHFD-NCD 031-2) administered with OLX-031-2 according to an embodiment. In particular, group 6 (CDHFD 16w 31-2) administered with OLX-031-2 had reduced levels of fat vacuole and collagen deposition compared to groups 3 and 4 (CDHFD 12w and CDHFD 16w) provided with a high-fat diet, which shows effective therapeutic efficacy of OLX-031-2 for nonalcoholic steatohepatitis.

### (2) Evaluation of expression of fibrosis-related factors in liver tissue

Expression levels of liver fibrosis-related factors were evaluated in the same manner as in (4) of Example 2-1.

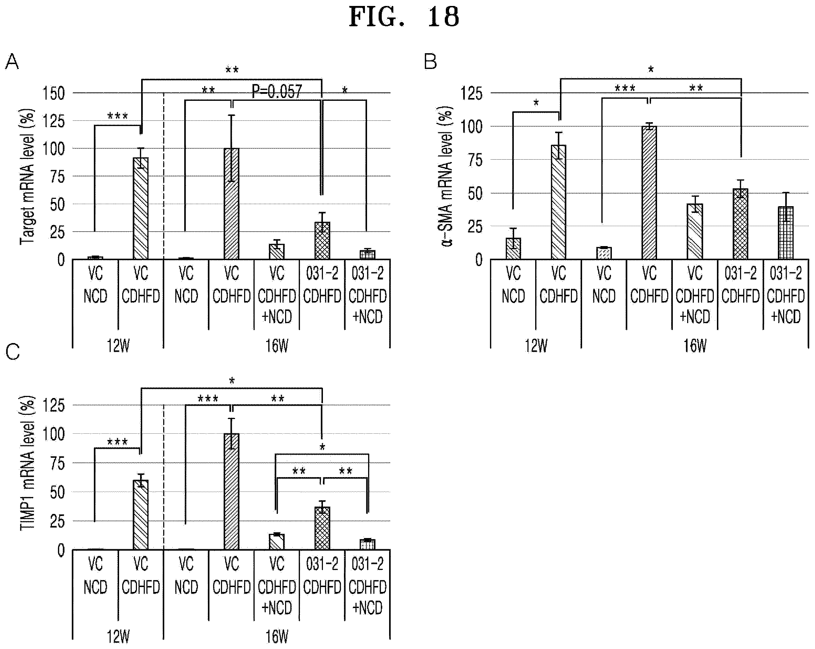

As a result, as shown in FIG. 18, it was confirmed that, expression levels of α-SMA, mCol1α1, and TIMP1 mRNA, which are factors related to liver fibrosis increased in groups provided with a high-fat diet (CDHFD and CDHFD-NCD), whereas increase in the expression of α-SMA, mCol1α1, and TIMP1 mRNA was decreased in groups administered with OLX-031-2 according to an example (#CDHFD 031-2 and CDHFD-NCD 031-2). Again, similar to the above-mentioned experimental results, group 6 (31-2 CDHFD) administered with OLX-031-2 showed a significant difference from groups 3 and 4 (CDHFD 12w and CDHFD 16w) that received a high-fat diet.

### (3) Evaluation of expression inhibitory effect on MARC1 mRNA

The liver tissue samples obtained from the mice were evaluated for expression levels of MARC1 mRNA in the same manner as in Example 1-2.

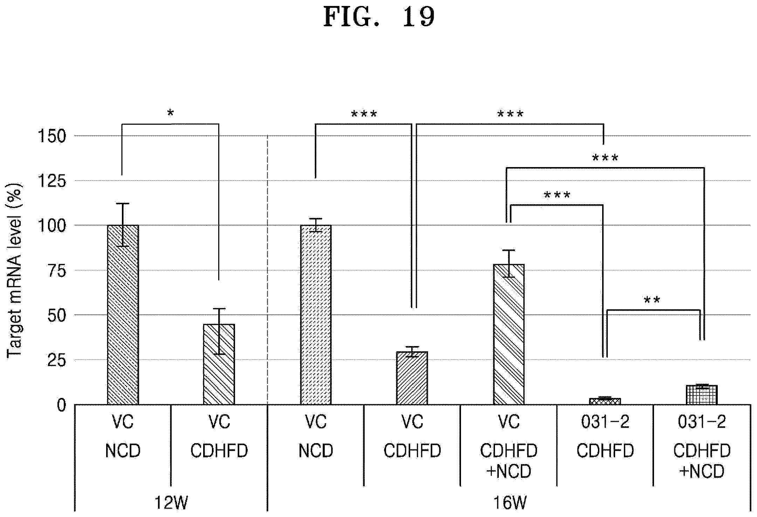

As a result, as shown in FIG. 19, in the groups (#CDHFD 031-2 and CDHFD-NCD 031-2) administered with OLX-031-2 according to an embodiment, expression levels of MARC1 mRNA were confirmed to be significantly lower than that of other groups.

### Example 3: Secondary screening of double-stranded nucleic acid molecules for RNAi induction

### 3-1. Design and preparation of 250 types of asiRNA

In this example, in the same manner as in 1-2 above, after obtaining information about MARC1 gene through NCBI database search, nucleotide sequences showing a certain level of homology were selected, based on the nucleotide sequences having a common target with each of MARC1 derived from a human and a monkey, and a total of 250 asiRNAs were designed (sense strand (16-mer), antisense strand (21-mer)) and the same were synthesized at 10 nmole scale by bioneer. Sequence information of the MARC1 asiRNA designed according to the above method is as shown in Tables 10 to 14 below.

**[Table 10]**

| **asiRNA name** | **Sequence(5' → 3')** | | **SEQ NO.** |
|---|---|---|---|
| asiMARC1-51 | sense | GAGGGAAACAUGGUUA | 101 |
| | antisense | UAACCAUGUUUCCCUCCUGGU | 102 |
| asiMARC1-52 | sense | GGAGGGAAACAUGGUA | 103 |
| | antisense | UACCAUGUUUCCCUCCUGGUU | 104 |
| asiMARC1-53 | sense | ACCAGGAGGGAAACAA | 105 |
| | antisense | UUGUUUCCCUCCUGGUUGAUC | 106 |
| asiMARC1-54 | sense | UCAACCAGGAGGGAAA | 107 |
| | antisense | UUUCCCUCCUGGUUGAUCACA | 108 |
| asiMARC1-55 | sense | GGCUUGUGAUCAACCA | 109 |
| | antisense | UGGUUGAUCACAAGCCAAAAC | 110 |
| asiMARC1-56 | sense | GUCCUGAUUUCCCUGA | 111 |
| | antisense | UCAGGGAAAUCAGGACCAGGC | 112 |
| asiMARC1-57 | sense | CUCGCCUGGUCCUGAA | 113 |
| | antisense | UUCAGGACCAGGCGAGGUUCC | 114 |
| asiMARC1-58 | sense | CCUCGCCUGGUCCUGA | 115 |
| | antisense | UCAGGACCAGGCGAGGUUCCU | 116 |
| asiMARC1-59 | sense | GACACCCUGACUCUCA | 117 |
| | antisense | UGAGAGUCAGGGUGUCACCAU | 118 |
| asiMARC1-60 | sense | UGGUGACACCCUGACA | 119 |
| | antisense | UGUCAGGGUGUCACCAUCGCA | 120 |
| asiMARC1-61 | sense | GCGAUGGUGACACCCA | 121 |
| | antisense | UGGGUGUCACCAUCGCAGGUC | 122 |
| asiMARC1-62 | sense | CCACCACAAAUGCAGA | 123 |
| | antisense | UCUGCAUUUGUGGUGGGCGUU | 124 |
| asiMARC1-63 | sense | GCCCACCACAAAUGCA | 125 |
| | antisense | UGCAUUUGUGGUGGGCGUUUU | 126 |
| asiMARC1-64 | sense | AACGCCCACCACAAAA | 127 |
| | antisense | UUUUGUGGUGGGCGUUUUGAU | 128 |
| asiMARC1-65 | sense | UGCACAAGUGCAGAGA | 129 |
| | antisense | UCUCUGCACUUGUGCACUGCA | 130 |
| asiMARC1-66 | sense | GCUUCCUGAAGUCACA | 131 |
| | antisense | UGUGACUUCAGGAAGCUGGUU | 132 |
| asiMARC1-67 | sense | CAGCUUCCUGAAGUCA | 133 |
| | antisense | UGACUUCAGGAAGCUGGUUAU | 134 |
| asiMARC1-68 | sense | ACCAGCUUCCUGAAGA | 135 |
| | antisense | UCUUCAGGAAGCUGGUUAUCC | 136 |
| asiMARC1-69 | sense | GCCCAGUGGAUAACCA | 137 |
| | antisense | UGGUUAUCCACUGGGCGGUGG | 138 |
| asiMARC1-70 | sense | ACCGCCCAGUGGAUAA | 139 |
| | antisense | UUAUCCACUGGGCGGUGGCCU | 140 |
| asiMARC1-71 | sense | CACUUCGAGCCUCACA | 141 |
| | antisense | UGUGAGGCUCGAAGUGCACCA | 142 |
| asiMARC1-72 | sense | UGCACUUCGAGCCUCA | 143 |
| | antisense | UGAGGCUCGAAGUGCACCAGG | 144 |
| asiMARC1-73 | sense | GACACCAGCCCAUUCA | 145 |
| | antisense | UGAAUGGGCUGGUGUCUGAGU | 146 |
| asiMARC1-74 | sense | CAGACACCAGCCCAUA | 147 |
| | antisense | UAUGGGCUGGUGUCUGAGUAA | 148 |
| asiMARC1-75 | sense | CCAGAUUGCUUACUCA | 149 |
| | antisense | UGAGUAAGCAAUCUGGUCCUU | 150 |
| asiMARC1-76 | sense | GACCAGAUUGCUUACA | 151 |
| | antisense | UGUAAGCAAUCUGGUCCUUGG | 152 |
| asiMARC1-77 | sense | AGGACCAGAUUGCUUA | 153 |
| | antisense | UAAGCAAUCUGGUCCUUGGGU | 154 |
| asiMARC1-78 | sense | ACCCAAGGACCAGAUA | 155 |
| | antisense | UAUCUGGUCCUUGGGUCGGAA | 156 |
| asiMARC1-79 | sense | GACCCAAGGACCAGAA | 157 |
| | antisense | UUCUGGUCCUUGGGUCGGAAC | 158 |
| asiMARC1-80 | sense | GCGAUGUCUAUGCAGA | 159 |
| | antisense | UCUGCAUAGACAUCGCAUCCU | 160 |
| asiMARC1-81 | sense | AGGAUGCGAUGUCUAA | 161 |
| | antisense | UUAGACAUCGCAUCCUGAAAU | 162 |
| asiMARC1-82 | sense | CAGGAUGCGAUGUCUA | 163 |
| | antisense | UAGACAUCGCAUCCUGAAAUU | 164 |
| asiMARC1-83 | sense | CCCAAUAUUGUAAUUA | 165 |
| | antisense | UAAUUACAAUAUUGGGCCUGA | 166 |
| asiMARC1-84 | sense | GCCCAAUAUUGUAAUA | 167 |
| | antisense | UAUUACAAUAUUGGGCCUGAA | 168 |
| asiMARC1-85 | sense | GGCCCAAUAUUGUAAA | 169 |
| | antisense | UUUACAAUAUUGGGCCUGAAG | 170 |
| asiMARC1-86 | sense | ACUUCAGGCCCAAUAA | 171 |
| | antisense | UUAUUGGGCCUGAAGUUGGUU | 172 |
| asiMARC1-87 | sense | AACUUCAGGCCCAAUA | 173 |
| | antisense | UAUUGGGCCUGAAGUUGGUUG | 174 |
| asiMARC1-88 | sense | CAACUUCAGGCCCAAA | 175 |
| | antisense | UUUGGGCCUGAAGUUGGUUGC | 176 |
| asiMARC1-89 | sense | CCAACUUCAGGCCCAA | 177 |
| | antisense | UUGGGCCUGAAGUUGGUUGCU | 178 |
| asiMARC1-90 | sense | AGAGAAGAAAGUUAAA | 179 |
| | antisense | UUUAACUUUCUUCUCUAGCCU | 180 |
| asiMARC1-91 | sense | UAGAGAAGAAAGUUAA | 181 |
| | antisense | UUAACUUUCUUCUCUAGCCUG | 182 |
| asiMARC1-92 | sense | CUAGAGAAGAAAGUUA | 183 |
| | antisense | UAACUUUCUUCUCUAGCCUGG | 184 |
| asiMARC1-93 | sense | CUGGCGGAUCUCAACA | 185 |
| | antisense | UGUUGAGAUCCGCCAGCGACG | 186 |
| asiMARC1-94 | sense | AGCUUCUUAUUGGUGA | 187 |
| | antisense | UCACCAAUAAGAAGCUCAUCC | 188 |
| asiMARC1-95 | sense | GCAUUUUAACCACAGA | 189 |
| | antisense | UCUGUGGUUAAAAUGCAUCUG | 190 |
| asiMARC1-96 | sense | UCCAGAUGCAUUUUAA | 191 |
| | antisense | UUAAAAUGCAUCUGGAACAAG | 192 |
| asiMARC1-97 | sense | UUCCAGAUGCAUUUUA | 193 |
| | antisense | UAAAAUGCAUCUGGAACAAGC | 194 |
| asiMARC1-98 | sense | GUUCCAGAUGCAUUUA | 195 |
| | antisense | UAAAUGCAUCUGGAACAAGCC | 196 |
| asiMARC1-99 | sense | UGUUCCAGAUGCAUUA | 197 |
| | antisense | UAAUGCAUCUGGAACAAGCCA | 198 |
| asiMARC1-100 | sense | UUGUUCCAGAUGCAUA | 199 |
| | antisense | UAUGCAUCUGGAACAAGCCAU | 200 |

**[Table 11]**

| **asiRNA name** | **Sequence(5' → 3')** | | **SEQ NO.** |
|---|---|---|---|
| asiMARC1-101 | sense | GUGUCAUGAGCAGGAA | 201 |
| | antisense | UUCCUGCUCAUGACACCGGUG | 202 |
| asiMARC1-102 | sense | CUGCUGGGCCAGUAAA | 203 |
| | antisense | UUUACUGGCCCAGCAGGUACA | 204 |
| asiMARC1-103 | sense | CCUGCUGGGCCAGUAA | 205 |
| | antisense | UUACUGGCCCAGCAGGUACAC | 206 |
| asiMARC1-104 | sense | CCCAGGGACCAUCAAA | 207 |
| | antisense | UUUGAUGGUCCCUGGGUUUUC | 208 |
| asiMARC1-105 | sense | ACCCAGGGACCAUCAA | 209 |
| | antisense | UUGAUGGUCCCUGGGUUUUCC | 210 |
| asiMARC1-106 | sense | GGCAGUAUUUUGUGCA | 211 |
| | antisense | UGCACAAAAUACUGCCCAAAG | 212 |
| asiMARC1-107 | sense | CUUUGGGCAGUAUUUA | 213 |
| | antisense | UAAAUACUGCCCAAAGAGUGG | 214 |
| asiMARC1-108 | sense | UCUUUGGGCAGUAUUA | 215 |
| | antisense | UAAUACUGCCCAAAGAGUGGU | 216 |
| asiMARC1-109 | sense | CUCUUUGGGCAGUAUA | 217 |
| | antisense | UAUACUGCCCAAAGAGUGGUG | 218 |
| asiMARC1-110 | sense | ACUCUUUGGGCAGUAA | 219 |
| | antisense | UUACUGCCCAAAGAGUGGUGA | 220 |
| asiMARC1-111 | sense | CACUCUUUGGGCAGUA | 221 |
| | antisense | UACUGCCCAAAGAGUGGUGAU | 222 |
| asiMARC1-112 | sense | CCACUCUUUGGGCAGA | 223 |
| | antisense | UCUGCCCAAAGAGUGGUGAUU | 224 |
| asiMARC1-113 | sense | GGAAAAUCACCACUCA | 225 |
| | antisense | UGAGUGGUGAUUUUCCAUAUA | 226 |
| asiMARC1-114 | sense | CGAAAGUUAUAUGGAA | 227 |
| | antisense | UUCCAUAUAACUUUCGUUCUG | 228 |
| asiMARC1-115 | sense | CAGAACGAAAGUUAUA | 229 |
| | antisense | UAUAACUUUCGUUCUGAAGGG | 230 |
| asiMARC1-116 | sense | UCAGAACGAAAGUUAA | 231 |
| | antisense | UUAACUUUCGUUCUGAAGGGU | 232 |
| asiMARC1-117 | sense | UUCAGAACGAAAGUUA | 233 |
| | antisense | UAACUUUCGUUCUGAAGGGUC | 234 |
| asiMARC1-118 | sense | CCUUCAGAACGAAAGA | 235 |
| | antisense | UCUUUCGUUCUGAAGGGUCAC | 236 |
| asiMARC1-119 | sense | ACCCUUCAGAACGAAA | 237 |
| | antisense | UUUCGUUCUGAAGGGUCACAC | 238 |
| asiMARC1-120 | sense | GACCCUUCAGAACGAA | 239 |
| | antisense | UUCGUUCUGAAGGGUCACACU | 240 |
| asiMARC1-121 | sense | GGAACCGCUGGAAACA | 241 |
| | antisense | UGUUUCCAGCGGUUCCUUCCU | 242 |
| asiMARC1-122 | sense | UCCUGGAAUAUUAGAA | 243 |
| | antisense | UUCUAAUAUUCCAGGACAUAC | 244 |
| asiMARC1-123 | sense | GUCCUGGAAUAUUAGA | 245 |
| | antisense | UCUAAUAUUCCAGGACAUACG | 246 |
| asiMARC1-124 | sense | AUGUCCUGGAAUAUUA | 247 |
| | antisense | UAAUAUUCCAGGACAUACGGU | 248 |
| asiMARC1-125 | sense | GUAUGUCCUGGAAUAA | 249 |
| | antisense | UUAUUCCAGGACAUACGGUUC | 250 |
| asiMARC1-126 | sense | CGUAUGUCCUGGAAUA | 251 |
| | antisense | UAUUCCAGGACAUACGGUUCC | 252 |
| asiMARC1-127 | sense | CCGUAUGUCCUGGAAA | 253 |
| | antisense | UUUCCAGGACAUACGGUUCCC | 254 |
| asiMARC1-128 | sense | ACCGUAUGUCCUGGAA | 255 |
| | antisense | UUCCAGGACAUACGGUUCCCA | 256 |
| asiMARC1-129 | sense | CAGAACUGAGACCUCA | 257 |
| | antisense | UGAGGUCUCAGUUCUGAAACA | 258 |
| asiMARC1-130 | sense | GGUGUUUCAGAACUGA | 259 |
| | antisense | UCAGUUCUGAAACACCAUGCU | 260 |
| asiMARC1-131 | sense | GCAUGGUGUUUCAGAA | 261 |
| | antisense | UUCUGAAACACCAUGCUUCAA | 262 |
| asiMARC1-132 | sense | AGCAUGGUGUUUCAGA | 263 |
| | antisense | UCUGAAACACCAUGCUUCAAG | 264 |
| asiMARC1-133 | sense | UUGAAGCAUGGUGUUA | 265 |
| | antisense | UAACACCAUGCUUCAAGUGUU | 266 |
| asiMARC1-134 | sense | CUCAAAAAUGACAACA | 267 |
| | antisense | UGUUGUCAUUUUUGAGAACAU | 268 |
| asiMARC1-135 | sense | CUCUACAUUUUCUUUA | 269 |
| | antisense | UAAAGAAAAUGUAGAGGUCUC | 270 |
| asiMARC1-136 | sense | CGUCUUUUGGACUUCA | 271 |
| | antisense | UGAAGUCCAAAAGACGAAAAA | 272 |
| asiMARC1-137 | sense | GCUUCAAUGUCCCAGA | 273 |
| | antisense | UCUGGGACAUUGAAGCAUUGA | 274 |
| asiMARC1-138 | sense | CUCAAUGCUUCAAUGA | 275 |
| | antisense | UCAUUGAAGCAUUGAGACACC | 276 |
| asiMARC1-139 | sense | GUCUCAAUGCUUCAAA | 277 |
| | antisense | UUUGAAGCAUUGAGACACCAG | 278 |
| asiMARC1-140 | sense | GUGUCUCAAUGCUUCA | 279 |
| | antisense | UGAAGCAUUGAGACACCAGAA | 280 |
| asiMARC1-141 | sense | UGGUGUCUCAAUGCUA | 281 |
| | antisense | UAGCAUUGAGACACCAGAAGU | 282 |
| asiMARC1-142 | sense | CAGGAUUCUGAAAACA | 283 |
| | antisense | UGUUUUCAGAAUCCUGUCUUG | 284 |
| asiMARC1-143 | sense | GACAGGAUUCUGAAAA | 285 |
| | antisense | UUUUCAGAAUCCUGUCUUGUC | 286 |
| asiMARC1-144 | sense | AGACAGGAUUCUGAAA | 287 |
| | antisense | UUUCAGAAUCCUGUCUUGUCA | 288 |
| asiMARC1-145 | sense | GACAAGACAGGAUUCA | 289 |
| | antisense | UGAAUCCUGUCUUGUCAUUUA | 290 |
| asiMARC1-146 | sense | AUGACAAGACAGGAUA | 291 |
| | antisense | UAUCCUGUCUUGUCAUUUAAG | 292 |
| asiMARC1-147 | sense | CCGUUUAACUGAUUAA | 293 |
| | antisense | UUAAUCAGUUAAACGGGGAGU | 294 |
| asiMARC1-148 | sense | CCCGUUUAACUGAUUA | 295 |
| | antisense | UAAUCAGUUAAACGGGGAGUU | 296 |
| asiMARC1-149 | sense | CCCCGUUUAACUGAUA | 297 |
| | antisense | UAUCAGUUAAACGGGGAGUUU | 298 |
| asiMARC1-150 | sense | CGUUUAUCUACCAAGA | 299 |
| | antisense | UCUUGGUAGAUAAACGGAGAA | 300 |

**[Table 12]**

| **asiRNA name** | **Sequence(5' → 3')** | | **SEQ NO.** |
|---|---|---|---|
| asiMARC1-151 | sense | UCCGUUUAUCUACCAA | 301 |
| | antisense | UUGGUAGAUAAACGGAGAAGC | 302 |
| asiMARC1-152 | sense | UCCUGUCACUACCACA | 303 |
| | antisense | UGUGGUAGUGACAGGAUGCAA | 304 |
| asiMARC1-153 | sense | GAGAAAGAGGAAGAGA | 305 |
| | antisense | UCUCUUCCUCUUUCUCUUCUU | 306 |
| asiMARC1-154 | sense | GAGAAGAAGAGAAAGA | 307 |
| | antisense | UCUUUCUCUUCUUCUCUUUCU | 308 |
| asiMARC1-155 | sense | AAGAGAAGAAGAGAAA | 309 |
| | antisense | UUUCUCUUCUUCUCUUUCUCU | 310 |
| asiMARC1-156 | sense | GAGAAAGAGAAGAAGA | 311 |
| | antisense | UCUUCUUCUCUUUCUCUAACG | 312 |
| asiMARC1-157 | sense | UACGCAAUGAAAAUUA | 313 |
| | antisense | UAAUUUUCAUUGCGUACCUCA | 314 |
| asiMARC1-158 | sense | GUACGCAAUGAAAAUA | 315 |
| | antisense | UAUUUUCAUUGCGUACCUCAU | 316 |
| asiMARC1-159 | sense | CAAAUAUGGCUGGAAA | 317 |
| | antisense | UUUCCAGCCAUAUUUGGGGUG | 318 |
| asiMARC1-160 | sense | CCCAAAUAUGGCUGGA | 319 |
| | antisense | UCCAGCCAUAUUUGGGGUGCA | 320 |
| asiMARC1-161 | sense | CUUUUGAAAUGGCAUA | 321 |
| | antisense | UAUGCCAUUUCAAAAGCUAGC | 322 |
| asiMARC1-162 | sense | GCUUUUGAAAUGGCAA | 323 |
| | antisense | UUGCCAUUUCAAAAGCUAGCC | 324 |
| asiMARC1-163 | sense | GGCUAGCUUUUGAAAA | 325 |
| | antisense | UUUUCAAAAGCUAGCCCGGGG | 326 |
| asiMARC1-164 | sense | GGGCUAGCUUUUGAAA | 327 |
| | antisense | UUUCAAAAGCUAGCCCGGGGC | 328 |
| asiMARC1-165 | sense | CGGGCUAGCUUUUGAA | 329 |
| | antisense | UUCAAAAGCUAGCCCGGGGCU | 330 |
| asiMARC1-166 | sense | CCGGGCUAGCUUUUGA | 331 |
| | antisense | UCAAAAGCUAGCCCGGGGCUU | 332 |
| asiMARC1-167 | sense | CCCCGGGCUAGCUUUA | 333 |
| | antisense | UAAAGCUAGCCCGGGGCUUGA | 334 |
| asiMARC1-168 | sense | GCCCCGGGCUAGCUUA | 335 |
| | antisense | UAAGCUAGCCCGGGGCUUGAG | 336 |
| asiMARC1-169 | sense | AGCCCCGGGCUAGCUA | 337 |
| | antisense | UAGCUAGCCCGGGGCUUGAGA | 338 |
| asiMARC1-170 | sense | CUCAAGCCCCGGGCUA | 339 |
| | antisense | UAGCCCGGGGCUUGAGAAGAA | 340 |
| asiMARC1-171 | sense | ACCUUCAGGAAGCACA | 341 |
| | antisense | UGUGCUUCCUGAAGGUCACCU | 342 |
| asiMARC1-172 | sense | AGGUGACCUUCAGGAA | 343 |
| | antisense | UUCCUGAAGGUCACCUCAGUC | 344 |
| asiMARC1-173 | sense | CCAUAGAUCUGGAUCA | 345 |
| | antisense | UGAUCCAGAUCUAUGGAAAAU | 346 |
| asiMARC1-174 | sense | CUGCAGAUAUUAAUUA | 347 |
| | antisense | UAAUUAAUAUCUGCAGUGCUU | 348 |
| asiMARC1-175 | sense | ACUGCAGAUAUUAAUA | 349 |
| | antisense | UAUUAAUAUCUGCAGUGCUUC | 350 |
| asiMARC1-176 | sense | CAUUGGAUUUCCUAAA | 351 |
| | antisense | UUUAGGAAAUCCAAUGCUGUC | 352 |
| asiMARC1-177 | sense | GCAUUGGAUUUCCUAA | 353 |
| | antisense | UUAGGAAAUCCAAUGCUGUCU | 354 |
| asiMARC1-178 | sense | AGCAUUGGAUUUCCUA | 355 |
| | antisense | UAGGAAAUCCAAUGCUGUCUG | 356 |
| asiMARC1-179 | sense | AGACAGCAUUGGAUUA | 357 |
| | antisense | UAAUCCAAUGCUGUCUGAGAA | 358 |
| asiMARC1-180 | sense | CAGACAGCAUUGGAUA | 359 |
| | antisense | UAUCCAAUGCUGUCUGAGAAG | 360 |
| asiMARC1-181 | sense | GCUUCUCAGACAGCAA | 361 |
| | antisense | UUGCUGUCUGAGAAGCAGCAG | 362 |
| asiMARC1-182 | sense | UGCUGCUUCUCAGACA | 363 |
| | antisense | UGUCUGAGAAGCAGCAGGGCC | 364 |
| asiMARC1-183 | sense | CCUGCUGCUUCUCAGA | 365 |
| | antisense | UCUGAGAAGCAGCAGGGCCAG | 366 |
| asiMARC1-184 | sense | AGGAUGGUUGUGUAGA | 367 |
| | antisense | UCUACACAACCAUCCUCCUGA | 368 |
| asiMARC1-185 | sense | CUGGAUCCUUGCCAUA | 369 |
| | antisense | UAUGGCAAGGAUCCAGGGGUC | 370 |
| asiMARC1-186 | sense | CCUGGAUCCUUGCCAA | 371 |
| | antisense | UUGGCAAGGAUCCAGGGGUCC | 372 |
| asiMARC1-187 | sense | CCCUGGAUCCUUGCCA | 373 |
| | antisense | UGGCAAGGAUCCAGGGGUCCU | 374 |
| asiMARC1-188 | sense | GGACCCCUGGAUCCUA | 375 |
| | antisense | UAGGAUCCAGGGGUCCUCCAU | 376 |
| asiMARC1-189 | sense | UGGAGGACCCCUGGAA | 377 |
| | antisense | UUCCAGGGGUCCUCCAUGACU | 378 |
| asiMARC1-190 | sense | UGCUCCUUCUCCAGUA | 379 |
| | antisense | UACUGGAGAAGGAGCACUCCG | 380 |
| asiMARC1-191 | sense | GUGCUCCUUCUCCAGA | 381 |
| | antisense | UCUGGAGAAGGAGCACUCCGU | 382 |
| asiMARC1-192 | sense | ACGGAGUGCUCCUUCA | 383 |
| | antisense | UGAAGGAGCACUCCGUCAUUA | 384 |
| asiMARC1-193 | sense | UGAAAAAGUUCUGAAA | 385 |
| | antisense | UUUCAGAACUUUUUCACCCGG | 386 |
| asiMARC1-194 | sense | GUGAAAAAGUUCUGAA | 387 |
| | antisense | UUCAGAACUUUUUCACCCGGA | 388 |
| asiMARC1-195 | sense | GGUGAAAAAGUUCUGA | 389 |
| | antisense | UCAGAACUUUUUCACCCGGAA | 390 |
| asiMARC1-196 | sense | CGGGUGAAAAAGUUCA | 391 |
| | antisense | UGAACUUUUUCACCCGGAACU | 392 |
| asiMARC1-197 | sense | UCCGGGUGAAAAAGUA | 393 |
| | antisense | UACUUUUUCACCCGGAACUGG | 394 |
| asiMARC1-198 | sense | AAGUGAUUCAGUGAUA | 395 |
| | antisense | UAUCACUGAAUCACUUUUCUU | 396 |
| asiMARC1-199 | sense | GAGAAGAAAAGUGAUA | 397 |
| | antisense | UAUCACUUUUCUUCUCCUCCA | 398 |
| asiMARC1-200 | sense | GGAGAAGAAAAGUGAA | 399 |
| | antisense | UUCACUUUUCUUCUCCUCCAC | 400 |

**[Table 13]**

| **asiRNA name** | **Sequence(5' → 3')** | | **SEQ NO.** |
|---|---|---|---|
| asiMARC1-201 | sense | GGAGGAGAAGAAAAGA | 401 |
| | antisense | UCUUUUCUUCUCCUCCACAGA | 402 |
| asiMARC1-202 | sense | GUGGAGGAGAAGAAAA | 403 |
| | antisense | UUUUCUUCUCCUCCACAGAAU | 404 |
| asiMARC1-203 | sense | UGUGGAGGAGAAGAAA | 405 |
| | antisense | UUUCUUCUCCUCCACAGAAUU | 406 |
| asiMARC1-204 | sense | CUGUGGAGGAGAAGAA | 407 |
| | antisense | UUCUUCUCCUCCACAGAAUUC | 408 |
| asiMARC1-205 | sense | GGGGAAAAGGAAAGCA | 409 |
| | antisense | UGCUUUCCUUUUCCCCCUUUA | 410 |
| asiMARC1-206 | sense | AAGGGGGAAAAGGAAA | 411 |
| | antisense | UUUCCUUUUCCCCCUUUAAAG | 412 |
| asiMARC1-207 | sense | CUUUAAAGGGGGAAAA | 413 |
| | antisense | UUUUCCCCCUUUAAAGGUUUU | 414 |
| asiMARC1-208 | sense | CCUUUAAAGGGGGAAA | 415 |
| | antisense | UUUCCCCCUUUAAAGGUUUUC | 416 |
| asiMARC1-209 | sense | ACCUUUAAAGGGGGAA | 417 |
| | antisense | UUCCCCCUUUAAAGGUUUUCA | 418 |
| asiMARC1-210 | sense | UACUGAAAACCUUUAA | 419 |
| | antisense | UUAAAGGUUUUCAGUAGUCUA | 420 |
| asiMARC1-211 | sense | CUACUGAAAACCUUUA | 421 |
| | antisense | UAAAGGUUUUCAGUAGUCUAU | 422 |
| asiMARC1-212 | sense | ACUACUGAAAACCUUA | 423 |
| | antisense | UAAGGUUUUCAGUAGUCUAUC | 424 |
| asiMARC1-213 | sense | GACUACUGAAAACCUA | 425 |
| | antisense | UAGGUUUUCAGUAGUCUAUCU | 426 |
| asiMARC1-214 | sense | UUGUUUAAAACCCAAA | 427 |
| | antisense | UUUGGGUUUUAAACAACUGAC | 428 |
| asiMARC1-215 | sense | CAUAUGUCAGUUGUUA | 429 |
| | antisense | UAACAACUGACAUAUGCUUUC | 430 |
| asiMARC1-216 | sense | GCAUAUGUCAGUUGUA | 431 |
| | antisense | UACAACUGACAUAUGCUUUCC | 432 |
| asiMARC1-217 | sense | AGCAUAUGUCAGUUGA | 433 |
| | antisense | UCAACUGACAUAUGCUUUCCU | 434 |
| asiMARC1-218 | sense | CCAUUUUGUCCUUUGA | 435 |
| | antisense | UCAAAGGACAAAAUGGCAAUA | 436 |
| asiMARC1-219 | sense | UGCCAUUUUGUCCUUA | 437 |
| | antisense | UAAGGACAAAAUGGCAAUAAA | 438 |
| asiMARC1-220 | sense | AGAUCUGAUGAAGUAA | 439 |
| | antisense | UUACUUCAUCAGAUCUUAGAG | 440 |
| asiMARC1-221 | sense | CUCUAAGAUCUGAUGA | 441 |
| | antisense | UCAUCAGAUCUUAGAGUUAUA | 442 |
| asiMARC1-222 | sense | GGAAGUUGACUAAACA | 443 |
| | antisense | UGUUUAGUCAACUUCCCAAUA | 444 |
| asiMARC1-223 | sense | UGGGAAGUUGACUAAA | 445 |
| | antisense | UUUAGUCAACUUCCCAAUAUA | 446 |
| asiMARC1-224 | sense | UUGGGAAGUUGACUAA | 447 |
| | antisense | UUAGUCAACUUCCCAAUAUAA | 448 |
| asiMARC1-225 | sense | CUACUUUGACUAGUUA | 449 |
| | antisense | UAACUAGUCAAAGUAGCUUCC | 450 |
| asiMARC1-226 | sense | GCUACUUUGACUAGUA | 451 |
| | antisense | UACUAGUCAAAGUAGCUUCCA | 452 |
| asiMARC1-227 | sense | AGCUACUUUGACUAGA | 453 |
| | antisense | UCUAGUCAAAGUAGCUUCCAU | 454 |
| asiMARC1-228 | sense | GGAAGCUACUUUGACA | 455 |
| | antisense | UGUCAAAGUAGCUUCCAUUUA | 456 |
| asiMARC1-229 | sense | GCCUGGUCCUGAUUUA | 457 |
| | antisense | UAAAUCAGGACCAGGCGAGGU | 458 |
| asiMARC1-230 | sense | CGCCUGGUCCUGAUUA | 459 |
| | antisense | UAAUCAGGACCAGGCGAGGUU | 460 |
| asiMARC1-231 | sense | UCGCCUGGUCCUGAUA | 461 |
| | antisense | UAUCAGGACCAGGCGAGGUUC | 462 |
| asiMARC1-232 | sense | CUGACUCUCAGUGCAA | 463 |
| | antisense | UUGCACUGAGAGUCAGGGUGU | 464 |
| asiMARC1-233 | sense | CCUGACUCUCAGUGCA | 465 |
| | antisense | UGCACUGAGAGUCAGGGUGUC | 466 |
| asiMARC1-234 | sense | CACCCUGACUCUCAGA | 467 |
| | antisense | UCUGAGAGUCAGGGUGUCACC | 468 |
| asiMARC1-235 | sense | GUGACACCCUGACUCA | 469 |
| | antisense | UGAGUCAGGGUGUCACCAUCG | 470 |
| asiMARC1-236 | sense | AGAUUGCUUACUCAGA | 471 |
| | antisense | UCUGAGUAAGCAAUCUGGUCC | 472 |
| asiMARC1-237 | sense | UCUCAACUCCAGGCUA | 473 |
| | antisense | UAGCCUGGAGUUGAGAUCCGC | 474 |
| asiMARC1-238 | sense | GGAUCUCAACUCCAGA | 475 |
| | antisense | UCUGGAGUUGAGAUCCGCCAG | 476 |
| asiMARC1-239 | sense | CGGAUCUCAACUCCAA | 477 |
| | antisense | UUGGAGUUGAGAUCCGCCAGC | 478 |
| asiMARC1-240 | sense | GCGGAUCUCAACUCCA | 479 |
| | antisense | UGGAGUUGAGAUCCGCCAGCG | 480 |
| asiMARC1-241 | sense | GGCGGAUCUCAACUCA | 481 |
| | antisense | UGAGUUGAGAUCCGCCAGCGA | 482 |
| asiMARC1-242 | sense | AGGGUGAUGGCUUGUA | 483 |
| | antisense | UACAAGCCAUCACCCUUUUCA | 484 |
| asiMARC1-243 | sense | ACCUGCUGGGCCAGUA | 485 |
| | antisense | UACUGGCCCAGCAGGUACACA | 486 |
| asiMARC1-244 | sense | AGAGAAAGAGAAGAAA | 487 |
| | antisense | UUUCUUCUCUUUCUCUAACGA | 488 |
| asiMARC1-245 | Sense | UAGAGAAAGAGAAGAA | 489 |
| | antisense | UUCUUCUCUUUCUCUAACGAG | 490 |
| asiMARC1-246 | Sense | AGGAGAAGAAAAGUGA | 491 |
| | antisense | UCACUUUUCUUCUCCUCCACA | 492 |
| asiMARC1-247 | sense | GCAGCCUACACAAAGA | 493 |
| | antisense | UCUUUGUGUAGGCUGCACUGA | 494 |
| asiMARC1-248 | sense | UGCAGCCUACACAAAA | 495 |
| | antisense | UUUUGUGUAGGCUGCACUGAG | 496 |
| asiMARC1-249 | sense | CAGUGCAGCCUACACA | 497 |
| | antisense | UGUGUAGGCUGCACUGAGAGU | 498 |
| asiMARC1-250 | sense | CUCAGUGCAGCCUACA | 499 |
| | antisense | UGUAGGCUGCACUGAGAGUCA | 500 |

**[Table 14]**

| **asiRNA name** | **Sequence(5' → 3')** | | **SEQ NO.** |
|---|---|---|---|
| asiMARC1-251 | sense | UCUCAGUGCAGCCUAA | 501 |
| | antisense | UUAGGCUGCACUGAGAGUCAG | 502 |
| asiMARC1-252 | sense | CUCUCAGUGCAGCCUA | 503 |
| | antisense | UAGGCUGCACUGAGAGUCAGG | 504 |
| asiMARC1-253 | sense | CCUUUCUGAGGCGUCA | 505 |
| | antisense | UGACGCCUCAGAAAGGAUCAA | 506 |
| asiMARC1-254 | sense | UCCUUUCUGAGGCGUA | 507 |
| | antisense | UACGCCUCAGAAAGGAUCAAG | 508 |
| asiMARC1-255 | sense | AAGAAAGUUAAAGCAA | 509 |
| | antisense | UUGCUUUAACUUUCUUCUCUA | 510 |
| asiMARC1-256 | sense | GGUGACGUGGAACUGA | 511 |
| | antisense | UCAGUUCCACGUCACCAAUAA | 512 |
| asiMARC1-257 | sense | UGGUGACGUGGAACUA | 513 |
| | antisense | UAGUUCCACGUCACCAAUAAG | 514 |
| asiMARC1-258 | sense | CAGAUGCAUUUUAACA | 515 |
| | antisense | UGUUAAAAUGCAUCUGGAACA | 516 |
| asiMARC1-259 | sense | CCAUCAAAGUGGGAGA | 517 |
| | antisense | UCUCCCACUUUGAUGGUCCCU | 518 |
| asiMARC1-260 | sense | CAGGGACCAUCAAAGA | 519 |
| | antisense | UCUUUGAUGGUCCCUGGGUUU | 520 |
| asiMARC1-261 | sense | CCAGGGACCAUCAAAA | 521 |
| | antisense | UUUUGAUGGUCCCUGGGUUUU | 522 |
| asiMARC1-262 | sense | UAUUUUGUGCUGGAAA | 523 |
| | antisense | UUUCCAGCACAAAAUACUGCC | 524 |
| asiMARC1-263 | sense | UUGGGCAGUAUUUUGA | 525 |
| | antisense | UCAAAAUACUGCCCAAAGAGU | 526 |
| asiMARC1-264 | sense | ACGCAAUGAAAAUUAA | 527 |
| | antisense | UUAAUUUUCAUUGCGUACCUC | 528 |
| asiMARC1-265 | sense | CCAAAUAUGGCUGGAA | 529 |
| | antisense | UUCCAGCCAUAUUUGGGGUGC | 530 |
| asiMARC1-266 | sense | CUUCUCAGACAGCAUA | 531 |
| | antisense | UAUGCUGUCUGAGAAGCAGCA | 532 |
| asiMARC1-267 | sense | GCUGCUUCUCAGACAA | 533 |
| | antisense | UUGUCUGAGAAGCAGCAGGGC | 534 |
| asiMARC1-268 | sense | AGGAGGGAAACAUGGA | 535 |
| | antisense | UCCAUGUUUCCCUCCUGGUUG | 536 |
| asiMARC1-269 | sense | CAGGAGGGAAACAUGA | 537 |
| | antisense | UCAUGUUUCCCUCCUGGUUGA | 538 |
| asiMARC1-270 | sense | CUGAUUUCCCUGACCA | 539 |
| | antisense | UGGUCAGGGAAAUCAGGACCA | 540 |
| asiMARC1-271 | sense | CCUGGUCCUGAUUUCA | 541 |
| | antisense | UGAAAUCAGGACCAGGCGAGG | 542 |
| asiMARC1-272 | sense | AGCCUACACAAAGGAA | 543 |
| | antisense | UUCCUUUGUGUAGGCUGCACU | 544 |
| asiMARC1-273 | sense | CAGCCUACACAAAGGA | 545 |
| | antisense | UCCUUUGUGUAGGCUGCACUG | 546 |
| asiMARC1-274 | sense | GUGCAGCCUACACAAA | 547 |
| | antisense | UUUGUGUAGGCUGCACUGAGA | 548 |
| asiMARC1-275 | sense | AGUGCAGCCUACACAA | 549 |
| | antisense | UUGUGUAGGCUGCACUGAGAG | 550 |
| asiMARC1-276 | sense | CCCUGACUCUCAGUGA | 551 |
| | antisense | UCACUGAGAGUCAGGGUGUCA | 552 |
| asiMARC1-277 | sense | ACCCUGACUCUCAGUA | 553 |
| | antisense | UACUGAGAGUCAGGGUGUCAC | 554 |
| asiMARC1-278 | sense | AAACGCCCACCACAAA | 555 |
| | antisense | UUUGUGGUGGGCGUUUUGAUA | 556 |
| asiMARC1-279 | sense | GCACAAGUGCAGAGUA | 557 |
| | antisense | UACUCUGCACUUGUGCACUGC | 558 |
| asiMARC1-280 | sense | GGUGCACUUCGAGCCA | 559 |
| | antisense | UGGCUCGAAGUGCACCAGGCG | 560 |
| asiMARC1-281 | sense | GCCUGGUGCACUUCGA | 561 |
| | antisense | UCGAAGUGCACCAGGCGGUAG | 562 |
| asiMARC1-282 | sense | CGCCUGGUGCACUUCA | 563 |
| | antisense | UGAAGUGCACCAGGCGGUAGG | 564 |
| asiMARC1-283 | sense | CCGCCUGGUGCACUUA | 565 |
| | antisense | UAAGUGCACCAGGCGGUAGGG | 566 |
| asiMARC1-284 | sense | ACCGCCUGGUGCACUA | 567 |
| | antisense | UAGUGCACCAGGCGGUAGGGC | 568 |
| asiMARC1-285 | sense | CGACCCAAGGACCAGA | 569 |
| | antisense | UCUGGUCCUUGGGUCGGAACA | 570 |
| asiMARC1-286 | sense | CCGACCCAAGGACCAA | 571 |
| | antisense | UUGGUCCUUGGGUCGGAACAA | 572 |
| asiMARC1-287 | sense | UGGCGGAUCUCAACUA | 573 |
| | antisense | UAGUUGAGAUCCGCCAGCGAC | 574 |
| asiMARC1-288 | sense | GUGACGUGGAACUGAA | 575 |
| | antisense | UUCAGUUCCACGUCACCAAUA | 576 |
| asiMARC1-289 | sense | UUGGUGACGUGGAACA | 577 |
| | antisense | UGUUCCACGUCACCAAUAAGA | 578 |
| asiMARC1-290 | sense | CCAGAUGCAUUUUAAA | 579 |
| | antisense | UUUAAAAUGCAUCUGGAACAA | 580 |
| asiMARC1-291 | sense | GGCCAGUAAUGGGAAA | 581 |
| | antisense | UUUCCCAUUACUGGCCCAGCA | 582 |
| asiMARC1-292 | sense | GGGCCAGUAAUGGGAA | 583 |
| | antisense | UUCCCAUUACUGGCCCAGCAG | 584 |
| asiMARC1-293 | sense | ACCAUCAAAGUGGGAA | 585 |
| | antisense | UUCCCACUUUGAUGGUCCCUG | 586 |
| asiMARC1-294 | sense | GACCAUCAAAGUGGGA | 587 |
| | antisense | UCCCACUUUGAUGGUCCCUGG | 588 |
| asiMARC1-295 | sense | UGGAAAACCCAGGGAA | 589 |
| | antisense | UUCCCUGGGUUUUCCAGCACA | 590 |
| asiMARC1-296 | sense | CUGGAAAACCCAGGGA | 591 |
| | antisense | UCCCUGGGUUUUCCAGCACAA | 592 |
| asiMARC1-297 | sense | CGCUGGAAACACUGAA | 593 |
| | antisense | UUCAGUGUUUCCAGCGGUUCC | 594 |
| asiMARC1-298 | sense | CCGCUGGAAACACUGA | 595 |
| | antisense | UCAGUGUUUCCAGCGGUUCCU | 596 |
| asiMARC1-299 | sense | ACCGCUGGAAACACUA | 597 |
| | antisense | UAGUGUUUCCAGCGGUUCCUU | 598 |
| asiMARC1-300 | sense | AACCGCUGGAAACACA | 599 |
| | antisense | UGUGUUUCCAGCGGUUCCUUC | 600 |

### 3-2. Evaluation of expression inhibitory effect on MARC1 mRNA

In order to confirm expression inhibitory efficiency at an mRNA level, the MARC1 asiRNA was transfected into Huh7 cells, and quantitative reverse transcription PCR (qRT-PCR) was performed to measure levels of MARC1 mRNA expression. Specifically, the Huh7 cells were seeded at 8×10³ cells/well in a 96 well plate, and MARC1 asiRNA (10 nM, OliX Inc.) and RNAiMax (2 µl/ml, Invitrogen Inc. 13778150) were added thereto, and transfection was performed according to the protocol provided by Invitrogen. 24 hours after the transfection, cell lysate was prepared using SuperPrep^{™} Cell lysis & RT kit for qPCR Kit II (TOYOBO, SCQ-401), and cDNA was synthesized through reverse transcription using the mRNA contained in the lysate as a template. Then, using the synthesized cDNA as a template, quantitative PCR was performed by using THUNDERBIRD^{®} Probe qPCR Mix (TOYOBO, QPS-101), and Probes (Hs00224227_m1, Hs03928985_g1, Applied Biosystems), and based on the mRNA expression inhibition level, a total of 134 MARC1 asiRNAs were primarily selected (not shown). Meanwhile, in this example, a group in which only a transfection reagent was added (Mock) was used as a control group, and a group in which transfection was performed in the same manner as in Example 1-2 using asiMARC1-30 was used as a positive control group (PC1). Then, for the 134 MARC1 asiRNAs selected as above, in the same manner as above, MARC1 mRNA expression levels were evaluated, while sequentially changing only the concentration of treated MARC1 asiRNA to 1 nM or 0.1 nM.

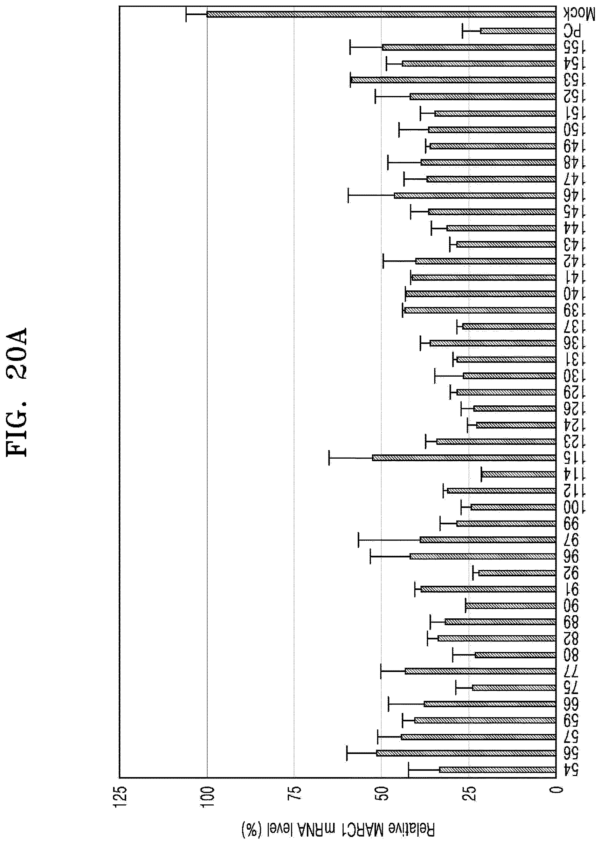

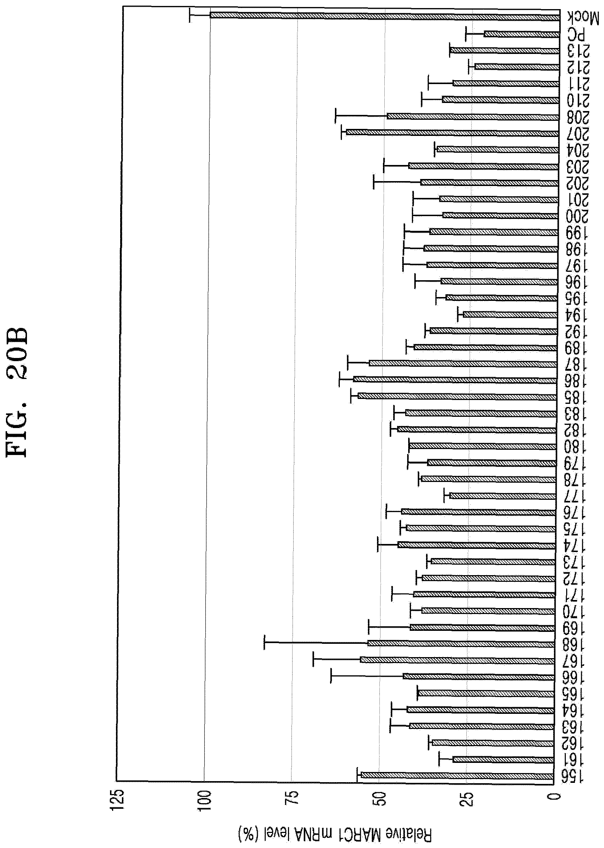

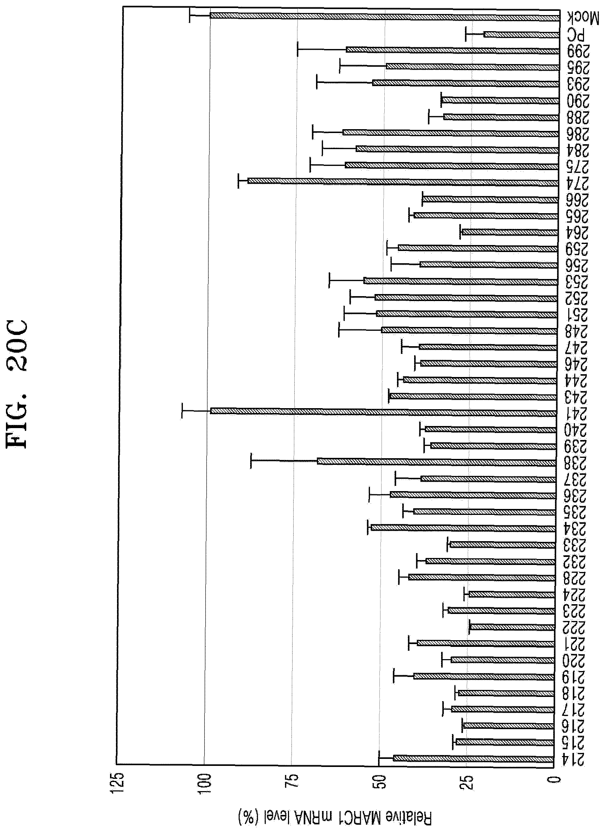

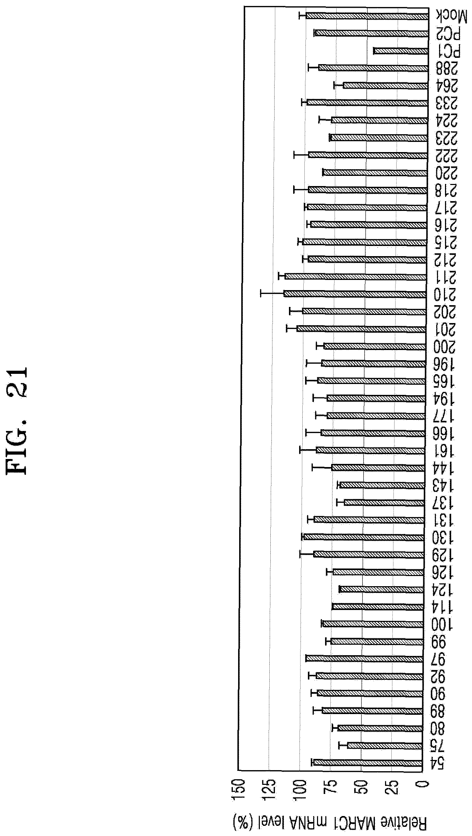

As a result, as shown in FIGS. 20A to 20C, the expression inhibitory effect on MARC1 mRNA by treatment of 1 nM of 134 types of MARC1 asiRNAs was confirmed, and among the 134 types of MARC1 asiRNAs, top 41 types of MARC1 asiRNAs with excellent MARC1 expression inhibitory efficiency were selected (#54, #75, #80, #89, #90, #92, #97, #99, #100, #114, #124, #126, #129, #130, #131, #137, #143, #144, #161, #166, #177, #194, #195, #196, #200, #201, #202, #210, #211, #212, #215, #216, #217, #218, #220, #222, #223, #224, #233, #264, #268). In addition, as shown in FIG. 21, the expression inhibitory effect on MARC1 mRNA by treatment of 0.1 nM of 41 types of MARC1 asiRNAs, in which expression inhibitory efficiency was confirmed, was confirmed, and among the 41 types of MARC1 asiRNAs, top 30 types of MARC1 asiRNAs having excellent MARC1 expression inhibitory efficiency were again selected (#75, #80, #89, #90, #92, #99, #100, #114, #124, #126, #129, #130, #131, #137, #143, #144, #166, #177, #194, #196, #200, #212, #215, #216, #218, #220, #222, #223, #224, #264).

### 3-3. Evaluation of expression inhibitory effect on MARC1 protein

In order to confirm expression inhibitory efficiency at a protein level, after transfection of 30 types of MARC1 asiRNAs selected in Example 3-2 into Huh7 cells, Western blot was performed to measure expression levels of MARC1 proteins. Specifically, the Huh7 cells were seeded at 5×10⁴ cells/well in a 12 well plate, and MARC1 asiRNA (1 nM, OliX Inc.) and RNAiMax (2 µl/ml, Invitrogen Inc. 13778150) were added thereto, and transfection was performed according to the protocol provided by Invitrogen. 48 hours after performing the transfection, the transfected cells were disrupted to obtain 15 µg of cell lysate for each sample. For the obtained cell lysate, Western blot was performed using 10 % SDS-polyacrylamide gel, MARC1 Rabbit polyclonal antibodies (1:1000 dilution in 3 % BSA, Abcepta, AP9754c), and Vinculin Mouse monoclonal antibodies (1:1000 dilution in 3 % BSA, Santa Cruz Biotechnology, sc-73614), and expression levels of MARC1 protein were confirmed by ChemiDoc XRS+ System (Bio-Rad). Meanwhile, in this example, a group in which only a transfection reagent was added (M) was used as a control group, and groups in which transfection was performed in the same manner as in Example 1-2 using asiMARC1-30 or asiMARC1-31 were used as positive control groups (PC1, PC2).

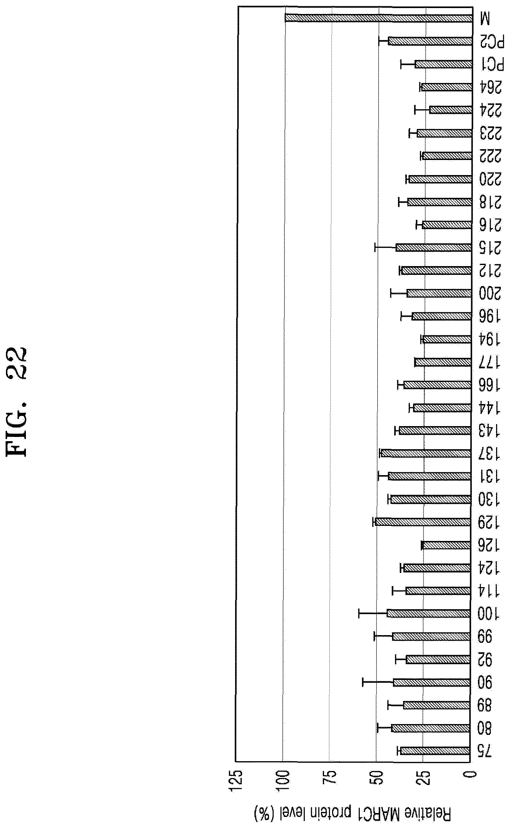

As a result, as shown in FIG. 22 , it was confirmed that all of the 30 types of MARC1 asiRNAs selected in Example 3-2 inhibited the expression of MARC1 protein.

### Example 4: Preparation of nucleic acid molecules for inducing RNAi with chemical modifications introduced

### 4-1. Design and preparation of 41 MARC1 GalNAc-asiRNAs

In this example, asymmetric siRNAs (GalNAc-asiRNAs) into which a GalNAc ligand and chemical modifications were introduced were designed for MARC1 asiRNAs, which were confirmed to have an expression inhibitory effect in Examples 1 and 3 above. The designed GalNAc-asiRNA is an asymmetric siRNA in which various chemical modifications (2'OMe, PS, Fluoro, GalNAc ligand, etc.) are introduced and delivery into hepatocytes is improved compared to the asiRNA.

Sequence information of a total of 41 MARC1 GalNAc-asiRNAs prepared in this example is shown in Table 15 below.

**[Table 15]**

| **GalNAc-asiRNA name** | **Sequence(5' → 3')** | |
|---|---|---|
| OLX-031-1 | sense | mG*fC*mGfCfAfGmCfUmCfUmGfGmAfUmCfU-GalNAc |
| | antisense | P-mA*fG*mAmUmCfCmAfGfAmGmCmUmGfCmG*fC*mC*mA*mC |
| OLX-031-3 | sense | mG*fC*mGfCfAfGmCfUmCfUmGfGmAfUmCfU-GalNAc |
| | antisense | P-mA*fG*mAmUmCmCfAmGfAmGmCmUmGfCmG*fC*mC*mA*mC |
| OLX-031-4 | sense | mG*fC*mGfCfAfGmCfUmCfUmGfGmAfUmCfA-GalNAc |
| | antisense | P-mU*fG*mAmUmCfCmAfGfAmGmCmUmGfCmG*fC*mC*mA*mC |
| OLX-031-6 | sense | mG*fC*mGfCfAfGmCfUmCfUmGfGmAfUmCfU-GalNAc |
| | antisense | P-mA*fG*mAmUmCfCmAfGfAmGmCmUmGfCmGfCmC*mA*mC*mU*mG |
| OLX-031-7 | sense | mG*fC*mGfCfAfGmCfUmCfUmGfGmAfUmCfU-GalNAc |
| | antisense | P-mA*fG*mAmUmCmCfAmGfAmGmCmUmGfCmGfCmC*mA*mC*mU*mG |
| OLX-031-8 | sense | mG*fC*mGfCfAfGmCfUmCfUmGfGmAfUmCfA-GalNAc |
| | antisense | P-mU*fG*mAmUmCfCmAfGfAmGmCmUmGfCmGfCmC*mA*mC*mU*mG |
| OLX-031-9 | sense | mG*fC*mGfCfAfGmCfUmCfUmGfGmAfUmCfA-GalNAc |
| | antisense | P-mU*fG*mAmUmCmCfAmGfAmGmCmUmGfCmGfCmC*mA*mC*mU*mG |
| OLX-075-1 | sense | mC*fC*mAfGfAfUmUfGmCfUmUfAmCfUmCfA-GalNAc |
| | antisense | P-mU*fG*mAmGmUmAfAmGfCmAmAmUmCfUmGfGmU*mC*mC*mU*mU |
| OLX-080-1 | sense | mG*fC*mGfAfUfGmUfCmUfAmUfGmCfAmGfA-GalNAc |
| | antisense | P-mU*fC*mUmGmCmAfUmAfGmAmCmAmUfCmGfCmA*mU*mC*mC*mU |
| OLX-089-1 | sense | mC*fC*mAfAfCfUmUfCmAfGmGfCmCfCmAfA-GaINAc |
| | antisense | P-mU*fU*mGmGmGmCfCmUfGmAmAmGmUfUmGfGmU*mU*mG*mC*mU |
| OLX-090-1 | sense | mA*fG*mAfGfAfAmGfAmAfAmGfUmUfAmAfA-GalNAc |
| | antisense | P-mU*fU*mUmAmAmCfUmUfUmCmUmUmCfUmCfUmA*mG*mC*mC*mU |
| OLX-092-1 | sense | mC*fU*mAfGfAfGmAfAmGfAmAfAmGfUmUfA-GalNAc |
| | antisense | P-mU*fA*mAmCmUmUfUmCfUmUmCmUmCfUmAfGmC*mC*mU*mG*mG |
| OLX-099-1 | sense | mU*fG*mUfUfCfCmAfGmAfUmGfCmAfUmUfA-GalNAc |
| | antisense | P-mU*fA*mAmUmGmCfAmUfCmUmGmGmAfAmCfAmA*mG*mC*mC*mA |
| OLX-100-1 | sense | mU*fU*mGfUfUfCmCfAmGfAmUfGmCfAmUfA-GalNAc |
| | antisense | P-mU*fA*mUmGmCmAfUmCfUmGmGmAmAfCmAfAmG*mC*mC*mA*mU |
| OLX-114-1 | sense | mC*fG*mAfAfAfGmUfUmAfUmAfUmGfGmAfA-GalNAc |
| | antisense | P-mU*fU*mCmCmAmUfAmUfAmAmCmUmUfUmCfGmU*mU*mC*mU*mG |
| OLX-124-1 | sense | mA*fU*mGfUfCfCmUfGmGfAmAfUmAfUmUfA-GalNAc |
| | antisense | P-mU*fA*mAmUmAmUfUmCfCmAmGmGmAfCmAfUmA*mC*mG*mG*mU |
| OLX-126-1 | sense | mC*fG*mUfAfUfGmUfCmCfUmGfGmAfAmUfA-GalNAc |
| | antisense | P-mU*fA*mUmUmCmCfAmGfGmAmCmAmUfAmCfGmG*mU*mU*mC*mC |
| OLX-129-1 | sense | mC*fA*mGfAfAfCmUfGmAfGmAfCmCfUmCfA-GalNAc |
| | antisense | P-mU*fG*mAmGmGmUfCmUfCmAmGmUmUfCmUfGmA*mA*mA*mC*mA |
| OLX-130-1 | sense | mG*fG*mUfGfUfUmUfCmAfGmAfAmCfUmGfA-GalNAc |
| | antisense | P-mU*fC*mAmGmUmUfCmUfGmAmAmAmCfAmCfCmA*mU*mG*mC*mU |
| OLX-131-1 | sense | mG*fC*mAfUfGfGmUfGmUfUmUfCmAfGmAfA-GalNAc |
| | antisense | P-mU*fU*mCmUmGmAfAmAfCmAmCmCmAfUmGfCmU*mU*mC*mA*mA |
| OLX-137-1 | sense | mG*fC*mUfUfCfAmAfUmGfUmCfCmCfAmGfA-GalNAc |
| | antisense | P-mU*fC*mUmGmGmGfAmCfAmUmUmGmAfAmGfCmA*mU*mU*mG*mA |
| OLX-143-1 | sense | mG*fA*mCfAfGfGmAfUmUfCmUfGmAfAmAfA-GalNAc |
| | antisense | P-mU*fU*mUmUmCmAfGmAfAmUmCmCmUfGmUfCmU*mU*mG*mU*mC |
| OLX-144-1 | sense | mA*fG*mAfCfAfGmGfAmUfUmCfUmGfAmAfA-GalNAc |
| | antisense | P-mU*fU*mUmCmAmGfAmAfUmCmCmUmGfUmCfUmU*mG*mU*mC*mA |
| OLX-166-1 | sense | mC*fC*mGfGfGfCmUfAmGfCmUfUmUfUmGfA-GalNAc |
| | antisense | P-mU*fC*mAmAmAmAfGmCfUmAmGmCmCfCmGfGmG*mG*mC*mU*mU |
| OLX-177-1 | sense | mG*fC*mAfUfUfGmGfAmUfUmUfCmCfUmAfA-GalNAc |
| | antisense | P-mU*fU*mAmGmGmAfAmAfUmCmCmAmAfUmGfCmU*mG*mU*mC*mU |
| OLX-194-1 | sense | mG*fU*mGfAfAfAmAfAmGfUmUfCmUfGmAfA-GaINAc |
| | antisense | P-mU*fU*mCmAmGmAfAmCfUmUmUmUmUfCmAfCmC*mC*mG*mG*mA |
| OLX-196-1 | sense | mC*fG*mGfGfUfGmAfAmAfAmAfGmUfUmCfA-GalNAc |
| | antisense | P-mU*fG*mAmAmCmUfUmUfUmUmCmAmCfCmCfGmG*mA*mA*mC*mU |
| OLX-200-1 | sense | mG*fG*mAfGfAfAmGfAmAfAmAfGmUfGmAfA-GaINAc |
| | antisense | P-mU*fU*mCmAmCmUfUmUfUmCmUmUmCfUmCfCmU*mC*mC*mA*mC |
| OLX-212-1 | sense | mA*fC*mUfAfCfUmGfAmAfAmAfCmCfUmUfA-GaINAc |
| | antisense | P-mU*fA*mAmGmGmUfUmUfUmCmAmGmUfAmGfUmC*mU*mA*mU*mC |
| OLX-216-1 | sense | mG*fC*mAfUfAfUmGfUmCfAmGfUmUfGmUfa-GalNAc |
| | antisense | P-mu*fA*mCmAmAmCfUmGfAmCmAmUmAfUmGfCmU*mU*mU*mC*mC |
| OLX-218-1 | sense | mC*fC*mAfUfUfUmUfGmUfCmCfUmUfUmGfA-GalNAc |
| | antisense | P-mU*fC*mAmAmAmGfGmAfCmAmAmAmAfUmGfGmC*mA*mA*mU*mA |
| OLX-220-1 | sense | mA*fG*mAfUfCfUmGfAmUfGmAfAmGfUmAfA-GalNAc |
| | antisense | P-mU*fU*mAmCmUmUfCmAfUmCmAmGmAfUmCfUmU*mA*mG*mA*mG |
| OLX-222-1 | sense | mG*fG*mAfAfGfUmUfGmAfCmUfAmAfAmCfA-GalNAc |
| | antisense | P-mU*fG*mUmUmUmAfGmUfCmAmAmCmUfUmCfCmC*mA*mA*mU*mA |
| OLX-223-1 | sense | mU*fG*mGfGfAfAmGfUmUfGmAfCmUfAmAfA-GalNAc |
| | antisense | P-mU*fU*mUmAmGmUfCmAfAmCmUmUmCfCmCfAmA*mU*mA*mU*mA |
| OLX-224-1 | sense | mU*fU*mGfGfGfAmAfGmUfUmGfAmCfUmAfA-GalNAc |
| | antisense | P-mU*fU*mAmGmUmCfAmAfCmUmUmCmCfCmAfAmU*mA*mU*mA*mA |
| OLX-233-1 | sense | mC*fC*mUfGfAfCmUfCmUfCmAfGmUfGmCfA-GaINAc |
| | antisense | P-mU*fG*mCmAmCmUfGmAfGmAmGmUmCfAmGfGmG*mU*mG*mU*mC |
| OLX-237-1 | sense | mU*fC*mUfCfAfAmCfUmCfCmAfGmGfCmUfA-GalNAc |
| | antisense | P-mU*fA*mGmCmCmUfGmGfAmGmUmUmGfAmGfAmU*mC*mC*mG*mC |
| OLX-264-1 | sense | mA*fC*mGfCfAfAmUfGmAfAmAfAmUfUmAfA-GalNAc |
| | antisense | P-mU*fU*mAmAmUmUfUmUfCmAmUmUmGfCmGfUmA*mC*mC*mU*mC |
| OLX-274-1 | sense | mG*fU*mGfCfAfGmCfCmUfAmCfAmCfAmAfA-GalNAc |
| | antisense | P-mU*fU*mUmGmUmGfUmAfGmGmCmUmGfCmAfCmU*mG*mA*mG*mA |
| OLX-276-1 | sense | mC*fC*mCfUfGfAmCfUmCfUmCfAmGfUmGfA-GaINAc |
| | antisense | P-mU*fC*mAmCmUmGfAmGfAmGmUmCmAfGmGfGmU*mG*mU*mC*mA |
| OLX-281-1 | sense | mG*fC*mCfUfGfGmUfGmCfAmCfUmUfCmGfA-GalNAc |
| | antisense | P-mU*fC*mGmAmAmGfUmGfCmAmCmCmAfGmGfCmG*mG*mU*mA*mG |

Specifically, chemical modifications denoted by "*", "m", "f", "P", and "GalNAc" in Table 15 are as shown in Table 7 above.

### 4-2. Evaluation of expression inhibitory effect on MARC1 mRNA

In order to confirm expression inhibition efficiencies at an mRNA level, primary cultured mouse hepatocytes (PMH) derived from C57BL/6 mice (Koatech) were treated (n=3) with a total of 41 types of MARC1 GalNAc-asiRNAs (100 nM) prepared in Example 4-1, and then, expression levels of MARC1 mRNA were measured by performing qRT-PCR in the same manner as in Example 1-2. On the other hand, in this example, a group not treated with a substance (NT) was used as a control group, a group treated with OLX700A-001-8 (100 nM) was used as a negative control group (NC), and a group treated with OLX-031-1 (10 nM) was used as a positive control group (PC).

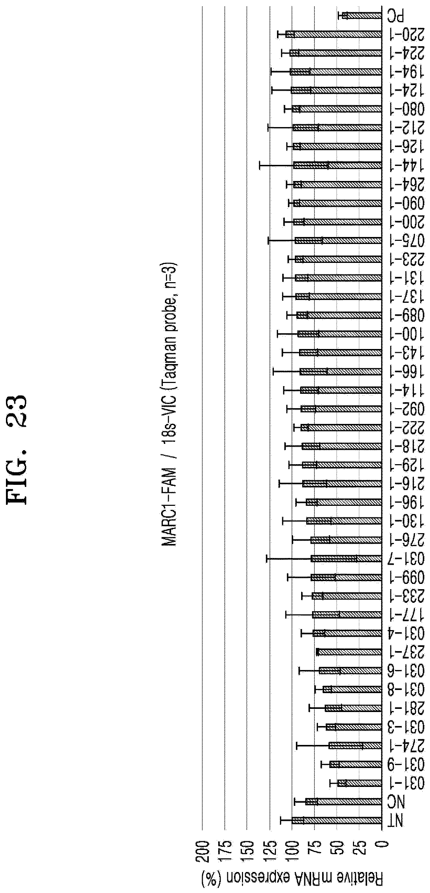

As a result, as shown in FIG. 23, it was confirmed that treatment of 41 types of MARC1 GalNAc-asiRNAs had expression inhibitory effect on MARC1 mRNA. Among these, expression inhibitory effects of OLX-031-1, OLX-031-9, OLX-274-1, OLX-231-3, OLX-281-1, OLX-031-8, OLX-031-6, OLX-237-1, and OLX-031-4 were more excellent.

### 4-3. Evaluation of expression inhibitory effect on MARC1 mRNA of human-derived hepatocytes

In order to identify expression inhibitory efficiency at an mRNA level, after treating (n=3), each of the 41 types of MARC1 GalNAc-asiRNAs prepared in Example 4-1 to primary human hepatocytes (F00995-P) derived from a human, qRT-PCR was performed to measure expression levels of MARC1 mRNA. Specifically, the primary hepatocytes were seeded in a 96-well plate at 4×10⁴ cells/well, treated with MARC1 GalNAc-asiRNA (500 nM, OliX Inc.), and then in the same manner as in Example 1-2, qRT-PCR was performed to measure expression levels of MARC1 mRNA. On the other hand, in this example, a group not treated with a substance (NT) was used as a control group, a group treated with OLX700A-001-8 (500 nM) was used as a negative control group (NC), and a group treated with OLX-031-1 (10 nM) was used as a positive control group (PC).

In addition, in order to confirm expression inhibitory efficiency according to the concentration of treated MARC1 GalNAc-asiRNA at a mRNA level, the human-derived primary hepatocytes were seeded at 3×10⁴ cells/well in a 96-well plate, and each were treated (n=2) with 20 nM or 100 nM of MARC1 GalNAc-asiRNA (OliX Inc.). Then, in the same manner as in Example 1-2, qRT-PCR was performed to measure expression levels of MARC1 mRNA. On the other hand, in this example, a group not treated with a substance (NT) was used as a control group, a group treated with OLX700A-001-8 (100 nM) was used as a negative control group (NC), and groups treated with OLX-075-1 or OLX-218-1 (10 nm)were used as positive control groups (PC1 and PC2).

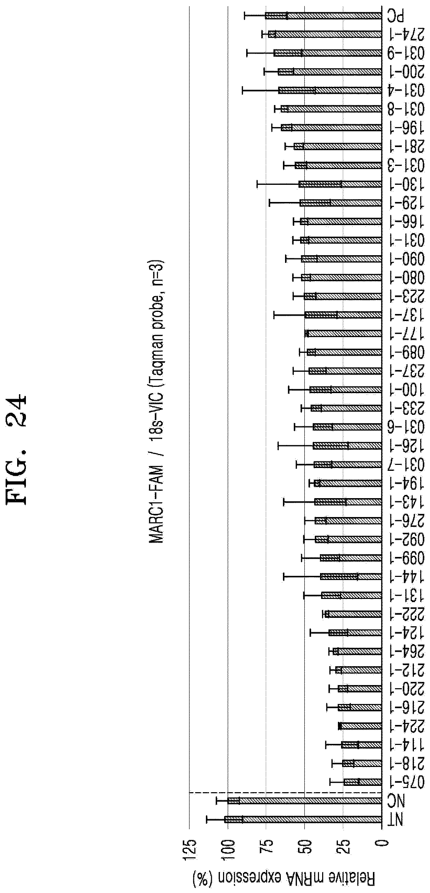

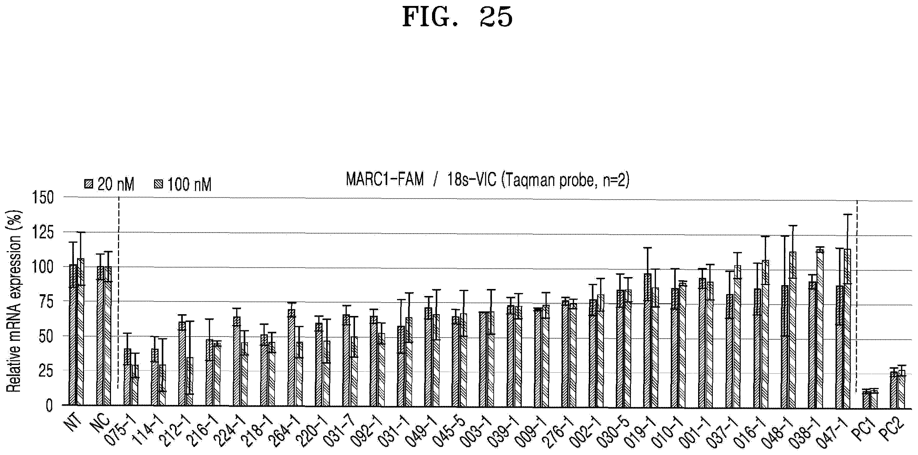

As a result, as shown in FIG. 24, expression inhibitory effect on MARC1 mRNA according to the treatment of 41 types of MARC1 GalNAc-asiRNAs was confirmed. In addition, as shown in FIG. 25 , the 41 types of MARC1 GalNAc-asiRNAs inhibited MARC1 expression in a concentration-dependent manner, and in particular, a total of 10 MARC1 GalNAc-asiRNAs having superior efficacy than OLX-031-1 obtained in Example 1 were selected (OLX-075-1, OLX-114-1, OLX-212-1, OLX-216-1, OLX-224-1, OLX-218-1, OLX-264-1, OLX-220-1, OLX-31-7, and OLX-92-1).

### Example 5. Preparation of GaINAc-asiRNA targeting human MARC1 and evaluation of expression inhibitory effect on MARC1 mRNA

In this example, MARC1 GalNAc-asiRNAs having various chemical modifications were prepared by selecting OLX-075-1, which showed an excellent effect, based on the experimental results of Example 4 above. Sequence information of a total of 17 MARC1 GalNAc-asiRNAs prepared in this example is shown in Table 16 below.

**[Table 16]**

| **GalNAc-asiRNA name** | **Sequence(5' → 3')** | |
|---|---|---|
| OLX-075-1 | sense | mC*fC*mAfGfAfUmUfGmCfUmUfAmCfUmCfA-GalNAc |
| | antisense | P-mU*fG*mAmGmUmAfAmGfCmAmAmUmCfUmGfGmU*mC*mC*mU*mU |
| OLX-075-2 | sense | mC*fC*mAfGfAf U m UfGmCf Um UfAmCfU mCfA-Gal NAc |
| | antisense | EVP-mU*fG*mAmGmUmAfAmGfCmAmAmUmCfUmGfGmU*mC*mC*mU*mU |
| OLX-075-3 | sense | mC*fC*mAfGfAfUmUfGmCfUmUfAmCfUmCfA-GalNAc |
| | antisense | EVP-mU*fG*mAmGmUmAmAmGmCmAmAmUmCfUmGfGmU*mC*mC*mU*mU |
| OLX-075-4 | sense | mC*fC*mAfGfAfUmUfGmCfUmUfAmCfUmCfA-GalNAc |
| | antisense | EVP-mU*fG*mAmGmUmAfAmGfCmAmAmUmCfUmG*fG*mU*mC*mC*mU*mU |
| OLX-075-5 | sense | mC*fC*mAfGfAfUmUfGmCfUmUfAmCfUmCfA-GalNAc |
| | antisense | EVP-mU*fG*mAmGmUmAmAmGmCmAmAmUmCfUmG*fG*mU*mC*mC*mU*mU |
| OLX-075-6 | sense | mC*fC*mAfGfAfUmUmGmCmUmUmAmCfUmCfA-GalNAc |
| | antisense | EVP-mU*fG*mAmGmUmAfAmGfCmAmAmUmCfUmGfGmU*mC*mC*mU*mU |
| OLX-075-7 | sense | mC*fC*mAfGfAfUmUmGmCmUmUmAmCfUmCfA-GalNAc |
| | antisense | EVP-mU*fG*mAmGmUmAmAmGmCmAmAmUmCfUmGfGmU*mC*mC*mU*mU |
| OLX-075-8 | sense | mC*fC*mAfGfAfUmUmGmCmUmUmAmCfUmCfA-GalNAc |
| | antisense | EVP-mU*fG*mAmGmUmAfAmGfCmAmAmUmCfUmG*fG*mU*mC*mC*mU*mU |
| OLX-075-9 | sense | mC*fC*mAfGfAfUmUmGmCmUmUmAmCfUmCfA-GalNAc |
| | antisense | EVP-mU*fG*mAmGmUmAmAmGmCmAmAmUmCfUmG*fG*mU*mC*mC*mU*mU |
| OLX-075-10 | sense | mC*fC*mAfGfGfUmGmGmCmCmUmAmCmUmCmA-GalNAc |
| | antisense | EVP-mU*fG*mAmGmUmAfAmGfCmAmAmUmCfUmGfGmU*mC*mC*mU*mU |
| OLX-075-11 | sense | mC*fC*mAfGfGfUmGmGmCmCmUmAmCmUmCmA-GalNAc |
| | antisense | EVP-mU*fG*mAmGmUmAmAmGmCmAmAmUmCfUmGfGmU*mC*mC*mU*mU |
| OLX-075-12 | sense | mC*fC*mAfGfGfUmGmGmCmCmUmAmCmUmCmA-GalNAc |
| | antisense | EVP-mU*fG*mAmGmUmAfAmGfCmAmAmUmCfUmG*fG*mU*mC*mC*mU*mU |
| OLX-075-13 | sense | mC*fC*mAfGfGfUmGmGmCmCmUmAmCmUmCmA-GalNAc |
| | antisense | EVP-mU*fG*mAmGmUmAmAmGmCmAmAmUmCfUmG*fG*mU*mC*mC*mU*mU |
| OLX-075-14 | sense | mC*mC*mAfGmAfUmUmGmCmUmUmAmCmUmCmA-GalNAc |
| | antisense | EVP-mU*fG*mAmGmUmAfAmGfCmAmAmUmCfUmGfGmU*mC*mC*mU*mU |
| OLX-075-15 | sense | mC*mC*mAfGmAfUmUmGmCmUmUmAmCmUmCmA-GaINAc |
| | antisense | EVP-mU*fG*mAmGmUmAmAmGmCmAmAmUmCfUmGfGmU*mC*mC*mU*mU |
| OLX-075-16 | sense | mC*mC*mAfGmAfUmUmGmCmUmUmAmCmUmCmA-GaINAc |
| | antisense | EVP-mU*fG*mAmGmUmAfAmGfCmAmAmUmCfUmG*fG*mU*mC*mC*mU*mU |
| OLX-075-17 | sense | mC*mC*mAfGmAfUmUmGmCmUmUmAmCmUmCmA-GaINAc |
| | antisense | EVP-mU*fG*mAmGmUmAmAmGmCmAmAmUmCfUmG*fG*mU*mC*mC*mU*mU |

Specifically, chemical modifications denoted by "*", "m", "f", "P", "GalNAc", and "EVP" in Table 16 are as shown in Table 7 above.

In addition, in order to identify expression inhibitory efficiency at an mRNA level, after treating (n=3), each of the 17 types of MARC1 GalNAc-asiRNA prepared above to primary human hepatocytes (F00995-P) derived from a human, qRT-PCR was performed to measure expression levels of MARC1 mRNA. Specifically, the primary hepatocytes were seeded in a 96-well plate at 4×10⁴ cells/well, treated with MARC1 GalNAc-asiRNA (10 nM or 100 nM, OliX Inc.), and then in the same manner as in Example 1-2, qRT-PCR was performed to measure expression levels of MARC1 mRNA.

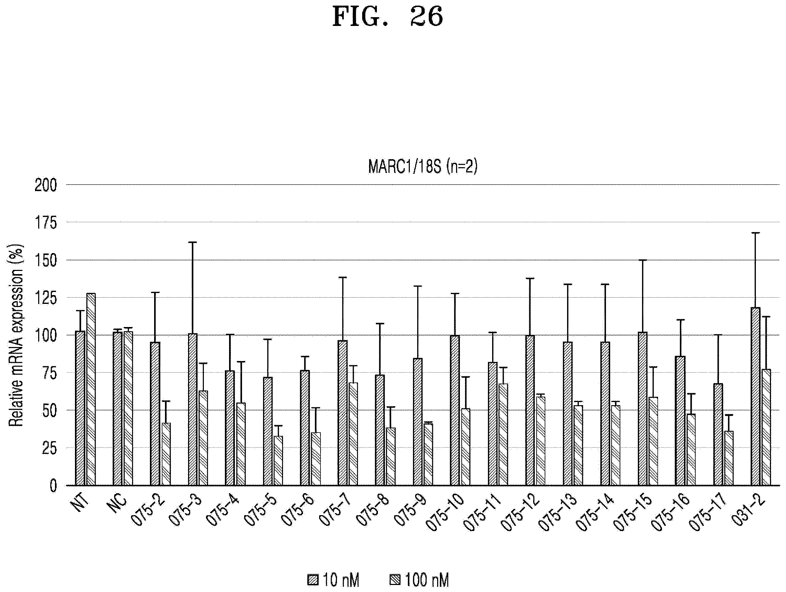

As a result, as shown in FIG. 26, expression inhibitory effect on MARC1 mRNA according to the treatment of 17 types of MARC1 GalNAc-asiRNAs was confirmed.

### Example 6. Evaluation of expression inhibitory effect on MARC1 mRNA using monkey models

Monkey animal models were subcutaneously administered with various concentrations (2.5 mp, 5 mp, or 10 mp) of OLX-031-2 or OLX-075-2 twice a week, and one week after the second administration was performed, the livers were extracted from the target monkeys, and liver tissue samples and sections thereof were obtained therefrom. Then, in the same manner as in Example 1-2, qRT-PCR was performed to measure expression levels of MARC1 mRNA. In this example, sequence information of OLX-031-2 and OLX-075-2 is as shown in Table 17 below, and specific classification of the animal model groups is as shown in Table 18 below.

**[Table 17]**

| **GaINAc-asiRNA name** | **Sequence(5' → 3')** | |
|---|---|---|
| OLX-031-2 | sense | mG*fC*mGfCfAfGmCfUmCfUmGfGmAfUmCfU-GalNAc |
| | antisense | EVP-mA*fG*mAmUmCfCmAfGfAmGmCmUmGfCmG*fC*mC*mA*mC |
| OLX-075-2 | sense | mC*fC*mAfGfAfUmUfGmCfUmUfAmCfUmCfA-GalNAc |
| | antisense | EVP-mU*fG*mAmGmUmAfAmGfCmAmAmUmCfUmGfGmU*mC*mC*mU*mU |

**[Table 18]**

| **Group** | **Dose** | **Treatment** | **Number** |
|---|---|---|---|
| 1 | 0 | VC (1×PBS) | 2 |
| 2 | 2.5 mpk | OLX-031-2 | 2 |
| 3 | 5 mpk | | 2 |
| 4 | 10 mpk | | 2 |
| 5 | 2.5 mpk | OLX-075-2 | 2 |
| 6 | 5 mpk | | 2 |
| 7 | 10 mpk | | 2 |

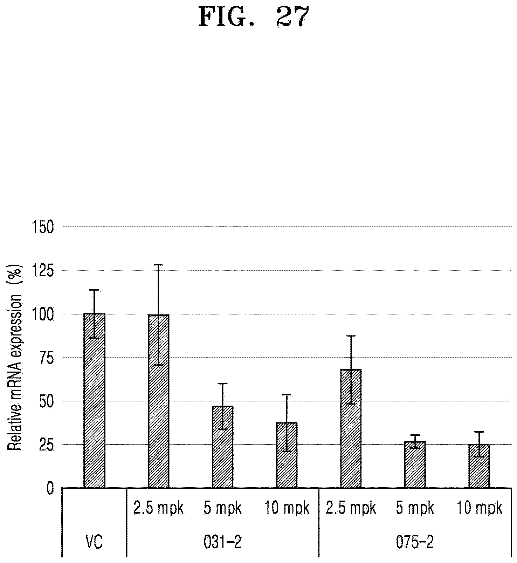

As a result, as shown in FIG. 27, a superior expression inhibitory effect on MARC1 mRNA was confirmed in the group administered with OLX-075-2, and this effect showed a concentration-dependent tendency.

### Example 7. Evaluation of expression inhibitory effect on MARC1 mRNA using animal models

### 7-1. Evaluation of expression inhibitory effect using SEAP reporter

After transfecting 6-week-old Balb/c mice (female) with a plasmid (pSELECT-mSEAP-hMARC1) containing a human MARC1 gene and a SEAP reporter linked thereto, the animal models were each divided into a group administered with 1X PBS (VC), a group administered with MARC1 GalNAc-asiRNA of Example 5 (OLX-075-2, OLX-075-4, OLX-075-5, OLX-075-6, OLX- 075-8, OLX-075-12, OLX-075-16, OLX-075-18) and a group administered with OLX-031-2 of Example 6, according to the substance administered. In this example, specific classification of the animal model groups is as shown in Table 19 below.

**[Table 19]**

| **Group** | **Dose** | **Treatment** | **Number** |
|---|---|---|---|
| 1 | 0 | VC (1×PBS) | 4 |
| 2 | 3 mpk | OLX-075-2 | 5 |
| 3 | 3 mpk | OLX-075-4 | 4 |
| 4 | 3 mpk | OLX-075-5 | 4 |
| 5 | 3 mpk | OLX-075-6 | 4 |
| 6 | 3 mpk | OLX-075-7 | 4 |
| 7 | 3 mpk | OLX-075-12 | 4 |
| 8 | 3 mpk | OLX-075-16 | 5 |
| 9 | 3 mpk | OLX-075-18 | 5 |
| 10 | 3 mpk | OLX-031-2 | 4 |

Meanwhile, MARC1 GalNAc-asiRNA was administered subcutaneously at 4 weeks from the time of transfection with the plasmid, and blood samples were collected 1 week after the administration and levels of SEAP reporter fluorescence was confirmed through a Phospha-Light^{™} SEAP Reporter Gene Assay System (Invitrogen^{™}, T1015), to compare expression inhibition levels on human MARC1 mRNA.

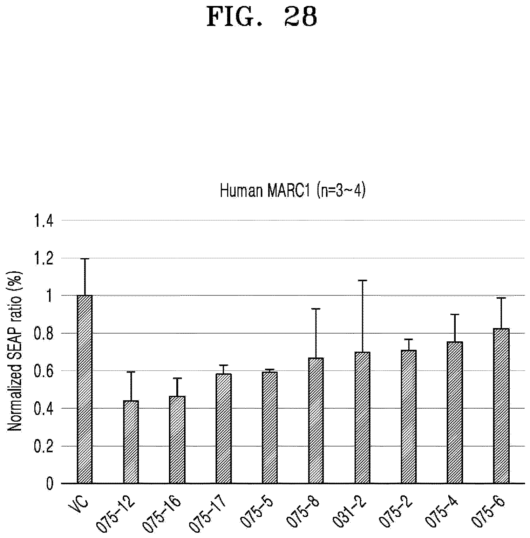

As a result, as shown in FIG. 28, the treatment of 9 types of MARC1 GalNAc-asiRNAs showed expression inhibitory effect on MARC1 mRNA, and among the samples, expression inhibitory effect in groups administered with OLX-075-12, OLX-075-16, OLX-075-17, OLX-075-5, OLX-075-8, or OLX-075-2 was more excellent.

### 7-2. Evaluation of expression inhibitory effect using dual luciferase reporter

After transfecting 6-week-old Balb/c mice with a plasmid containing a human MARC1 gene (psiCHECK-2-hMARC1), 3mpk of MARC1 GalNAc-asiRNAs of Example 5 were subcutaneously administered (OLX-075-2, OLX-075-4, OLX-075-5, OLX-075-6, OLX-075-8, OLX-075- 12, OLX-075-16, OLX-075-17, OLX-075-18). Three days after the administration, blood samples were taken, and levels of Luciferase reporter fluorescence were identified through a Dual-Luciferase^{®} Reporter Assay System (Promega, E1980) to compare expression inhibition levels on human MARC1 mRNA.

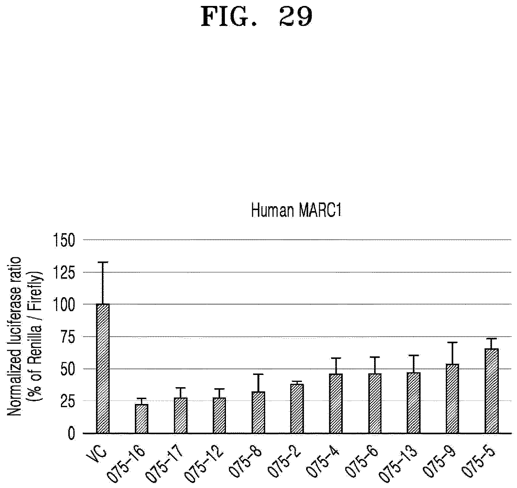

As a result, as shown in FIG. 29, the groups administered with MARC1 GalNAc-asiRNAs of Example 5 having various chemical modifications all showed excellent expression inhibitory effect on MARC1 mRNA, and among the samples, expression inhibitory effect of the groups administered with OLX-075-16, OLX-075-17, OLX-075-12, OLX-075-8, OLX-075-8 was more excellent.

Thus far, specific portions of the content of the present disclosure have been described in detail, and it will be apparent to those of ordinary skill in the art that these specific descriptions are only preferred embodiments, and that the scope of the present disclosure is not limited thereby. Accordingly, it is intended that the substantial scope of the present disclosure is defined by the appended claims and their equivalents.

## Claims

1. An RNAi agent comprising: an antisense strand having sequence complementarity to the mRNA sequence of mitochondrial amidoxime reducing component 1 (MARC1), and is 19 nt to 21 nt in length (nt); and a sense strand having sequence complementarity to the antisense strand, and is 15 nt to 17 nt in length, wherein the 5' end of the antisense strand and the 3' end of the sense strand form a blunt end.

2. The RNAi agent of claim 1, wherein the sense strand has a sequence selected from the sense strand sequences listed in Table 1, Table 10, Table 11, Table 12, Table 13, and Table 14.

3. The RNAi agent of claim 1, wherein the sense strand is any one selected from the group consisting of SEQ ID NO: 149, SEQ ID NO: 159, SEQ ID NO: 177, SEQ ID NO: 179, SEQ ID NO: 183, SEQ ID NO: 197, SEQ ID NO: 199, SEQ ID NO: 227, SEQ ID NO: 247, SEQ ID NO: 251, SEQ ID NO: 257, SEQ ID NO: 259, SEQ ID NO: 261, SEQ ID NO: 273, SEQ ID NO: 285, SEQ ID NO: 287, SEQ ID NO: 331, SEQ ID NO: 353, SEQ ID NO: 387, SEQ ID NO: 391, SEQ ID NO: 399, SEQ ID NO: 423 , SEQ ID NO: 429, SEQ ID NO: 431, SEQ ID NO: 435, SEQ ID NO: 439, SEQ ID NO: 443, SEQ ID NO: 445, SEQ ID NO: 447, and SEQ ID NO: 527.

4. The RNAi agent of claim 1, wherein the sense strand is any one selected from the group consisting of SEQ ID NO: 61, SEQ ID NO: 149, SEQ ID NO: 183, SEQ ID NO: 227, SEQ ID NO: 423, SEQ ID NO: 431, SEQ ID NO: 435, SEQ ID NO: 439, SEQ ID NO: 447, SEQ ID NO: 527, and SEQ ID NO: 551.

5. The RNAi agent of claim 1, wherein the antisense strand has a sequence selected from the antisense strand sequences listed in Table 1, Table 10, Table 11, Table 12, Table 13, and Table 14.

6. The RNAi agent of claim 1, wherein the antisense strand is any one selected from the group consisting of SEQ ID NO: 150, SEQ ID NO: 160, SEQ ID NO: 178, SEQ ID NO: 180, SEQ ID NO: 184, SEQ ID NO: 198, SEQ ID NO: 200, SEQ ID NO: 228, SEQ ID NO: 248, SEQ ID NO: 252, SEQ ID NO: 258, SEQ ID NO: 260, SEQ ID NO: 262, SEQ ID NO: 274, SEQ ID NO: 286, SEQ ID NO: 288, SEQ ID NO: 332, SEQ ID NO: 354, SEQ ID NO: 388, SEQ ID NO: 392, SEQ ID NO: 400, SEQ ID NO: 424, SEQ ID NO: 430, SEQ ID NO: 432, SEQ ID NO: 436, SEQ ID NO: 440, SEQ ID NO: 444, SEQ ID NO: 446, SEQ ID NO: 448, and SEQ ID NO: 528.

7. The RNAi agent of claim 1, wherein the antisense strand is any one selected from the group consisting of SEQ ID NO: 62, SEQ ID NO: 150, SEQ ID NO: 184, SEQ ID NO: 228, SEQ ID NO: 424, SEQ ID NO: 432, SEQ ID NO: 436, SEQ ID NO: 440, SEQ ID NO: 448, SEQ ID NO: 528, and SEQ ID NO: 552.

8. The RNAi agent of claim 1, wherein the RNAi agent inhibits expression of MARC1.

9. The RNAi agent of claim 1, wherein the sense strand is bound to an N-acetylgalactosamine (GalNAc) derivative at the 3' end.

10. The RNAi agent of claim 1, wherein the sense strand or the antisense strand comprises at least one chemical modification.

11. The RNAi agent of claim 10, wherein the sense strand comprises at least one chemical modification selected from:
modification of 2 to 4 nucleotide bonds adjacent to the 5' end with phosphorothioate, boranophosphate, or methyl phosphonate;
substitution of an -OH group at the 2' carbon position of the sugar structure of at least one nucleotide with -CH₃ (methyl), -OCH₃ (methoxy), -NH₂, -F, -O-2-methoxyethyl-O-propyl, -O-2-methyl methylthioethyl, -O-3-aminopropyl, or -O-3-dimethylaminopropyl;
and binding with an N-acetylgalactosamine (GalNAc) derivative, or a cell penetrating peptide at the 3' end.

12. The RNAi agent of claim 10, wherein the antisense strand comprises at least one chemical modification selected from:
modification of 2 to 7 nucleotide bonds adjacent to the 3' end or the 5' end with phosphorothioate, boranophosphate, or methyl phosphonate;
substitution of an -OH group at the 2' carbon position of the sugar structure of at least one nucleotide with -CH₃ (methyl), -OCH₃ (methoxy), -NH₂, -F, -O-2-methoxyethyl-O-propyl, -O-2-methyl methylthioethyl, -O-3-aminopropyl, or -O-3-dimethylaminopropyl;
and binding with a phosphate group, E-vinylphosphonate, or a cell penetrating peptide at the 5' end.

13. The RNAi agent of claim 10, further comprising at least one modification selected from the group consisting of:
modification of 2 to 7 nucleotide bonds adjacent to the 3' end or the 5' end in a sense or an antisense strand to phosphorothioate;
modification of an -OH group at the 2' carbon position of the sugar structure of at least one nucleotide in a sense strand or an antisense strand to -OCH₃ (methoxy), or -F;
binding to an N-acetylgalactosamine (GalNAc) derivative at the 3' end of the sense strand; and
binding with a phosphate group, or E-vinylphosphonate at the 5' end of an antisense strand.

14. The RNAi agent of claim 10, wherein the sense strand has a sequence selected from the sense strand sequences listed in Table 2, Table 15, Table 16, and Table 17.

15. The RNAi agent of claim 10, wherein the antisense strand has a sequence selected from the antisense strand sequences listed in Table 2, Table 15, Table 16, and Table 17.

16. A pharmaceutical composition for preventing or treating liver disease, comprising the RNAi agent according to any one of claims 1 to 15 as an active ingredient.

17. The pharmaceutical composition for preventing or treating liver disease of claim 16, wherein the liver disease is fatty liver, liver fibrosis, or cirrhosis.

18. The pharmaceutical composition for preventing or treating liver disease of claim 17, wherein the fatty liver is non-alcoholic fatty liver disease (NAFLD).

19. The pharmaceutical composition for preventing or treating liver disease of claim 18, wherein the non-alcoholic fatty liver disease is simple steatosis or non-alcoholic steatohepatitis (NASH).

20. A method of preventing or treating liver disease, comprising administering to a subject the RNAi agent according to any one of claims 1 to 15.

21. A use of the RNAi agent according to any one of claims 1 to 15 for manufacturing a pharmaceutical product for preventing or treating liver disease.
